# EUROPEAN PATENT SPECIFICATION

(11) **EP 4 362 873 B1**
(45) Date of publication and mention of the grant of the patent: **05.08.2026**
(21) Application number: 23738904.4
(22) Date of filing: 29.06.2023
(51) Int. Cl.: A61F 13/0246, A61F 13/05, A61F 13/00

(54) **WOUND CLOSURE SYSTEMS, DEVICES AND METHODS INCORPORATING THERAPEUTIC DEVICES**
WUNDVERSCHLUSSSYSTEME, VORRICHTUNGEN UND VERFAHREN MIT THERAPEUTISCHEN VORRICHTUNGEN
SYSTÈMES, DISPOSITIFS ET PROCÉDÉS DE FERMETURE DE PLAIE INCORPORANT DES DISPOSITIFS THÉRAPEUTIQUES

(30) Priority: 30.06.2022 US 202217854923
(43) Date of publication of application: 08.05.2024
(73) Proprietor: Cilag GmbH International, 6300 Zug (CH)
(72) Inventor: BICK, Alison, Raritan, New Jersey 08869 (US); QUINTERO, Julian, Raritan, New Jersey 08869 (US); SYBY, Jonathan, Raritan, New Jersey 08869 (US); KRIKSUNOV, Leo, Raritan, New Jersey 08869 (US)
(74) Representative: Carpmaels & Ransford LLP
(86) International application number: PCT/IB2023/056758
(87) International publication number: WO 2024/003817

(56) References cited:
- JP-A- 2021 532 880
- US-A1- 2019 076 140

## Description

### BACKGROUND OF THE INVENTION

### Field of the Invention

The present patent application is generally related to surgical procedures, and is more specifically related to systems, devices and kits for closing wounds and promoting efficient healing of wounds.

### Description of the Related Art

During a surgery, a surgical incision is a cut made through the skin and/or soft tissue to facilitate an operation or procedure. In many instances, multiple incisions may be necessary. In general, a surgical incision is made as small and unobtrusive as possible to facilitate safe and timely operating conditions.

At the end of a surgical procedure, the surgical incisions must be closed. Over the years, many different techniques have been developed for closing surgical incisions. One widely used technique involves using sutures having a single knot or a series of knots. In recent years, absorbable sutures and barbed sutures have been used for closing surgical incisions. In addition, surgical tacks have been used for closing surgical incisions, which reduces the amount of time required to close the surgical incisions.

Other system for closing wounds and incisions involve the use of mesh tapes that are adhered to skin. With these systems, skin or tissue parts separated by the incision are approximated or brought into close proximity by forming as narrow a gap as possible in the area of the surgical incision. The approximate incision is then covered by an adhesively attached mesh tape, which holds the skin or tissue in closed apposed arrangement until wound healing occurs after which the tape is removed.

For example, a skin closure system sold under the trademark DERMABOND^{®} PRINEO^{®} Skin Closure System by Ethicon, Inc. of Somerville, New Jersey, includes a mesh having a pressure sensitive adhesive and a polymerization initiator disposed on the mesh. The mesh is applied onto the skin over a wound, and a polymerizable cyanoacrylate-based adhesive is then applied on the mesh and bonds the mesh to the skin.

Another technical advance directed to closing surgical incisions without using sutures or surgical tacks is disclosed in commonly assigned U.S. Pat. No. 8,777,986 to Straehnz et al.. The '986 patent discloses an incision guide and wound closure device including a surgical mesh having a top surface and a bottom surface, and first and second incision guides affixed to the top surface of the surgical mesh. The bottom surface of the mesh is adhered to tissue using clear or translucent adhesive. The first and second incision guides have opposing alignment surfaces that are adapted to guide a cutting instrument for making an incision through the mesh and into the tissue. The device has a closing element that is moveable along the length of the respective first and second incision guides for drawing the first and second alignment surfaces toward one another for closing the incision opening in the tissue.

U.S. Patent Application Publication No. 2015/0314114, discloses a device adapted for dressing and treating wounds, skin lesions/ulcers, sores and burns, comprising at least one biocompatible membrane and at least one catheter coupled to the membrane.

U.S. Patent Application Publication No. 2014/0121649 discloses a surgical wound dressing assembly for a surgical tube wound or other device entrance into the body, comprising a cover layer, with adhesive on the outer perimeter of the underside of the cover layer, an absorbent ring secured to the underside of the cover, an interior clear window allowing the nurse or other medical personnel to view the wound site through the window for inspection for infection or bleeding, and a port formed in the window area to allow a tube or other device to pass through the wound dressing.

U.S. Patent No. 10,993,708 to Quintero et al., assigned to Ethicon, Inc. of Somerville, New Jersey, teaches a skin closure device having interrupted closure. The skin closure device includes a flat porous mesh that is elongated along a longitudinal axis, and that has an upper side, a lower wound facing side, and a central portion in the immediate vicinity of the longitudinal axis. The flat porous mesh has a plurality of pores and windows formed in the flat porous mesh. The windows are substantially larger than the pores and are arranged along the longitudinal axis in the central portion. A cross-linking or polymerization accelerator or initiator is disposed in or on the mesh, and a pressure sensitive adhesive disposed on at least a portion of the lower surface of the mesh.

Due to various comorbidities, some patients may require wound closure systems, some may require negative pressure wound therapy, some may require close monitoring using sensors, and others may require wound drainage, etc.

There is no single solution, however, that allows for surgical wound approximation, closure, controlled removal of exudate, maintenance of closure over the healing period, not requiring dressing changes, and allowing negative pressure to be applied controllably.

Thus, there remains a need for improved wound closure systems that may be used on patients suffering from a broad array of conditions. There also remains a need for a universal, customizable and/or highly versatile wound closure system that can be applied to wounds and accommodate additional modalities (e.g., a negative pressure wound therapy device; a sensor), or no additional modality, if none is required.

### SUMMARY OF THE INVENTION

Patients with significant comorbidities typically require advanced wound closure approaches including appropriate skin tissue approximation/closure and negative pressure to minimize post-surgical complications and delays during recovery. The present invention relates to wound closure systems and kits described in the appended claims. Optional features are recited in the dependent claims.

In one embodiment, a wound closure system preferably allows for surgical wound approximation, skin closure, exudate removal, maintenance of closure over the healing period, not requiring dressing changes, and/or allowing negative pressure to be applied controllably.

In one embodiment, a wound closure system preferably provides for controlled wound drainage, wound irrigation, wound cleansing, antimicrobial irrigation, pain medication administration, etc.

The wound closure system includes a porous mesh having a pressure sensitive adhesive (PSA) applied on both a bottom, skin-facing surface used for wound approximation and a top surface configured for securing one or more therapeutic devices, if desired.

The pressure sensitive adhesive is applied to both the top and bottom surfaces of the porous mesh.

The pressure sensitive adhesive is covered by liners (e.g., liner paper) that are removed and/or peeled away to expose the pressure sensitive adhesive.

The pressure sensitive adhesive applied to the bottom, skin-facing surface of the porous mesh is stronger or more densely applied than the pressure sensitive adhesive applied to the top surface of the porous mesh.

In one embodiment, a polymerization catalyst or accelerator may be disposed in or on the porous mesh for promoting or enhancing curing of a liquid adhesive.

The wound closure system (also referred to herein as a skin closure system) preferably includes a porous mesh that is coated on both sides with a pressure sensitive adhesive. The pressure sensitive adhesive applied to the bottom, skin-facing surface is used to approximate or close wound edges (also referred to herein as skin edges), while the pressure sensitive adhesive applied to the top surface of the porous mesh provides a sticky surface (due to the PSA) that allows for the placement (with opportunity to reposition) one or more therapeutic devices on top of the porous mesh, such as drainage tubes that may be connected to negative pressure pumps for draining wound exudate. The therapeutic devices may also allow for the post-operative introduction of medications, or allow sensors to be placed on the porous mesh for monitoring the wound. Once the one or more therapeutic devices are in place and the wound is approximated, a rapidly polymerizable or cross-linkable liquid adhesive may be applied over the deployed porous mesh and the one or more therapeutic devices secured atop the porous mesh.

When cured, the liquid adhesive preferably forms an air-tight or waterproof seal around the approximated wound or incision.

In one embodiment not recited in the appended claims, a method of using a wound closure system may include removing the first liner from the skin-facing side of the mesh, and positioning the mesh with the skin-facing side on a wound for approximating the wound.

In one embodiment not recited in the appended claims, a method may include removing the second liner from the opposite, top surface of the mesh, and positioning a therapeutic device (e.g., a negative pressure wound therapy device; a sensor; a medical device; an accessory, etc.) over the top surface of the mesh.

In one embodiment, after initial placement atop the mesh, the therapeutic device may be pulled away from the mesh and repositioned one or more times over the top surface of the mesh. It may be necessary to reposition a therapeutic device to achieve an optimal or better therapeutic benefit.

In one embodiment, a rapidly polymerizable adhesive may be applied on top of the mesh and on top of at least a portion of the therapeutic device.

In one embodiment, the adhesive is allowed to polymerize for binding the porous mesh to skin and/or the wound, and to further bind the therapeutic device to the porous mesh.

In one embodiment, a wound closure system preferably includes an adhesive mesh that is placed on an incision to close the incision. In one embodiment, both sides of the adhesive mesh have a pressure sensitive adhesive applied thereto. One side of the adhesive mesh is in intimate contact with the skin and is used to approximate wound edges, and the other side of the adhesive mesh has pressure sensitive adhesive used to precisely place tubing and other accessories (e.g., a sensor; a negative pressure wound therapy device) at the incision line.

In one embodiment, a drain tube having holes and/or slits may be placed in contact with the top surface of the mesh for adhering to the pressure sensitive adhesive applied to the top surface of the mesh. In one embodiment, the drain tube may have two ends, a first end coupled with a vacuum for draining exudate, and a second end including an inlet port for injecting fluids, saline, or medication, which may be infused over the approximated wound or incision. A primary function of the drain tube is to drain exudate from the incision.

The above-described wound closure system is highly customizable, whereby medical personnel may adjust the length of the drain tube, as necessary, and may choose which types of therapeutic devices should be secured to the top surface of the mesh (e.g., choose which type of sensors are required to monitor the approximated incision).

In one embodiment, a topical skin adhesive may be applied over the top of a therapeutic device (e.g., an elongated tube) that is secured to the top surface of the mesh. The tubing and therapeutic devices (e.g., sensors) may be aligned with the incision line. Once cured, the adhesive preferably forms a strong, air-tight seal around the incision. The adhesive may also form a waterproof and/or antimicrobial barrier, and may further enable medical personnel to monitor the wound healing process. The topical skin adhesive may be any rapidly polymerizable or cross-linkable, biocompatible adhesive known to those skilled in the art. In one embodiment, the topical skin adhesive may be a cyanoacrylate-based adhesive, such as the 2-Octyl cyanoacrylate adhesive sold under the trademark DERMABOND^{®} Skin Adhesive - Topical Skin Glue by Ethicon, Inc. of Somerville, New Jersey. Alternatively, the adhesive system may be sold under the trademark DERMABOND^{®} PRINEO^{®} - Skin Closure System, by Ethicon, Inc. of Somerville, New Jersey. In certain embodiments, some cyanoacrylate-based adhesives may work in concert with an accelerator or a catalyst of polymerization that is disposed on the mesh. Alternatively, the topical skin adhesive may be, for example, a two-part silicone-based skin or tissue adhesive, such as those disclosed in US 2021/0371658 and US 2021/0369639, assigned to Ethicon, Inc. of Somerville, New Jersey. Other silicone-based adhesive systems are disclosed, for example, in U.S. Patent Nos. 10,533,074 and 11,161,937, assigned to Ethicon, Inc. of Somerville, New Jersey. In one embodiment, a silicone adhesive may include a composition comprising a cross-linkable silicone polymer having reactive functionalities; a silica-containing composition; a silicone cross-linking agent; and a catalyst. In one embodiment, the catalyst comprises a platinum tetramethyldivinyl disiloxane diethyl maleate complex. The cross-linkable silicone polymer can be selected from the group consisting of vinyl terminated polydialkylsiloxane, vinyl terminated polydimethylsiloxane, vinyl terminated polydiphenylsilane-dimethylsiloxane copolymer, vinyl terminated polyphenylmethylsiloxane, vinyl terminated polyfluoropropylmethyl-dimethylsiloxane copolymer, vinyl terminated polydiethylsiloxane, and SiH terminated polydimethyldisiloxane. In one embodiment, the silica-containing composition may include a trimethyl silyl surface treated silica filler. The silicone cross-linking agent may be selected from the group consisting of polymethylhydrosiloxane, polymethylhydro-co-polydimethylsiloxane, polyethyhydrosiloxane, polymethylhydrosiloxane-co-octylmethylsiloxane, and polymethylhydrosiloxane-co-methylphenylsiloxane.

The wound closure system includes a porous mesh having a top surface and a bottom surface, a first pressure sensitive adhesive applied to the top surface of the porous mesh, a removable first liner covering the first pressure sensitive adhesive, a second pressure sensitive adhesive applied to the bottom surface of the porous mesh, and a removable second liner covering the second pressure sensitive adhesive.

The second pressure sensitive adhesive is stronger or denser than the first pressure sensitive adhesive. In one embodiment, the second pressure sensitive adhesive has greater bonding strength than the first pressure sensitive adhesive. In one embodiment, the first and second pressure sensitive adhesives may have the same bonding strength, however, a greater mass or volume of the second pressure sensitive adhesive is applied to the bottom surface of the porous mesh relative to the mass/volume of the first pressure sensitive adhesive applied to the top surface of the porous mesh.

In one embodiment, a cross-linking or polymerization accelerator or catalyst is preferably disposed in or on the porous mesh.

In one embodiment, the porous mesh is flat, elongated along a longitudinal axis, and has a plurality of openings (e.g., pores) extending between the top and bottom surfaces thereof.

In one embodiment, the removable first liner is configured to be peeled away from the top surface of the porous mesh for exposing the first pressure sensitive adhesive.

In one embodiment, the removable second liner is configured to be peeled away from the bottom surface of the porous mesh for exposing the second pressure sensitive adhesive.

In one embodiment, a method of using the wound closure system disclosed herein may include peeling the removable second liner away from the bottom surface of the porous mesh to expose the second pressure sensitive adhesive applied to the bottom surface of the porous mesh, and pressing the second pressure sensitive adhesive applied to the bottom surface of the porous mesh against skin of a patient to approximate opposing edges of a wound.

In one embodiment not recited in the appended claims, the method may include peeling the removable first liner away from the top surface of the porous mesh to expose the first pressure sensitive adhesive applied to the top surface of the porous mesh, and pressing a therapeutic device against the first pressure sensitive adhesive to secure the therapeutic device over the top surface of the porous mesh.

In one embodiment not recited in the appended claims, the method may include applying a liquid adhesive over the top surface of the porous mesh and at least a portion of the therapeutic device, and allowing the liquid adhesive to cure for binding the porous mesh to the skin and binding the therapeutic device to the porous mesh.

In one embodiment, the liquid adhesive preferably includes a rapidly polymerizable or cross-linkable liquid adhesive.

In one embodiment, the therapeutic device is aligned with the wound prior to being pressed against the top surface of the porous mesh. In one embodiment, if the therapeutic device is not properly aligned with the wound, it may be lifted away from the top surface of the porous mesh and repositioned one or more times until the therapeutic device is in alignment with the wound.

In one embodiment, the therapeutic device may be utilized for draining exudate from the wound or infusing saline or medication into the wound.

The therapeutic device may be a medical device including but not limited to negative pressure wound therapy devices, negative pressure pumps, fluid suction devices, elongated tubes including drainage tubes and infusion tubes, elongated tubes having first and second mesh wings extending along lateral sides thereof, cannulas, wound monitoring sensors, cameras, and combinations thereof.

The kit for treating wounds includes a wound closure system having a porous mesh with a top surface and a bottom surface, a first pressure sensitive adhesive applied to the top surface of the porous mesh, a removable first liner covering the first pressure sensitive adhesive, a second pressure sensitive adhesive applied to the bottom surface of the porous mesh, and a removable second liner covering the second pressure sensitive adhesive, the second pressure sensitive adhesive being stronger or denser than the first pressure sensitive adhesive.

The kit includes a rapidly polymerizable liquid adhesive disposed within a dispenser container.

The kit includes one or more therapeutic devices configured to be secured over the top surface of the porous mesh. The one or more therapeutic devices may include negative pressure wound therapy devices, negative pressure pumps, fluid suction devices, elongated tubes including drainage tubes and infusion tubes, elongated tubes having first and second mesh wings extending along lateral sides thereof, cannulas, and wound monitoring sensors.

In one embodiment, a cross-linking or polymerization accelerator or catalyst may be disposed in or on the porous mesh for interacting with the rapidly polymerizable liquid adhesive disposed within a dispenser container.

In one embodiment, a wound closure system desirably includes a porous mesh having a top surface and a bottom surface, the bottom surface of the porous mesh being adhered to skin for approximating opposing edges of a wound, and a therapeutic device including an elongated tube secured to the top surface of the porous mesh and overlying the wound, the elongated tube being configured for draining exudate from the wound.

In one embodiment, the wound closure system preferably includes a liquid adhesive covering the top surface of the porous mesh and at least a portion of the elongated tube.

In one embodiment, the therapeutic device my include first and second mesh wings secured to the elongated tube and extending along respective lateral sides of the elongated tube.

In one embodiment, the liquid adhesive preferably covers the first and second mesh wings for securing the therapeutic device to the top surface of the porous mesh.

In one embodiment, the elongated tube may include a first elongated conduit that is coupled with a vacuum or negative pressure pump for draining the exudate from the wound.

In one embodiment, the elongated tube may include a second elongated conduit that is coupled with a positive pressure pump, which is configured to force saline or medication into the elongated tube for infusing the wound with the saline or the medication.

In one embodiment, the liquid adhesive may be a topical skin adhesive or a rapidly polymerizable adhesive that forms an air-tight seal around the wound.

In one embodiment, the air-tight seal formed by the liquid adhesive may be waterproof or may include an antimicrobial barrier.

In one embodiment, a cross-linking or polymerization accelerator or catalyst may be disposed in or on the porous mesh. The cross-linking or polymerization accelerator or catalyst preferably interacts with the liquid adhesive applied to the mesh for rapidly curing the adhesive.

In one embodiment, a wound closure system may be affixed over a wound and remain in place for about 7- 14 days

In one embodiment, during treatment of a wound, a therapeutic device, after being adhered atop a mesh, may be removed from the mesh.

In one embodiment, during treatment of a wound, a first therapeutic device may be removed from being secured atop the mesh and replaced with a second therapeutic device that is secured atop the mesh. The first and second therapeutic devices may have different functions and/or capabilities.

In one embodiment, a wound closure system may include one or more drain tubes having different lengths, whereby the one or more drain tubes may be customized for tailoring the length of a drain tube to a length of a wound or incision.

In one embodiment, a drain tube may be clear or partially transparent for enabling medical personnel to view the wound or incision located below the drain tube, which may assist medical personnel in aligning the drain tube with the wound or incision.

In one embodiment, the drain tube may be made of silicone.

In one embodiment, pressure sensitive adhesive may be applied directly to a therapeutic device (e.g., an elongated tube, a drain tube, a negative pressure wound therapy device, a sensor, etc.) so that the therapeutic device may be adhered to a mesh or skin adjacent approximated wound edges or approximated skin edges.

In one embodiment, a liquid adhesive or topical skin adhesive applied over a therapeutic device may have anti-microbial properties, anti-clotting properties, anti-fouling properties and/or anti-clogging properties.

In one embodiment, a wound closure system may include a first layer of a porous dressing (e.g., a first mesh; a bottom mesh) having a top surface and a bottom surface. In one embodiment, the top surface and/or the bottom surface of the first layer of the porous dressing may be tacky and/or have an adhesive (e.g., a pressure sensitive adhesive) disposed thereon.

In one embodiment, the first layer of the porous dressing may include a bottom surface that is tacky or that has an adhesive (e.g., a pressure sensitive adhesive) applied thereto for securing (e.g., adhering) the first layer of the porous dressing to tissue (e.g., skin).

In one embodiment, a therapeutic device (e.g., a drain; a suction device; a sensor) may be positioned over the top surface of the first layer of the porous dressing. In one embodiment, the therapeutic device may have a surface that is tacky or that has an adhesive applied thereto for attaching the therapeutic device to the top surface of the first layer of the porous dressing.

In one embodiment, the therapeutic device may have an elongated channel that is configured for draining a wound or incision. In one embodiment, the bottom surface of the therapeutic device may have one or more fenestrations formed therein that are in communication with the elongated channel and that are positioned against the top surface of the first layer of the porous dressing. In one embodiment, the therapeutic device has a bottom surface that is tacky and/or has an adhesive provided thereon for securing the bottom surface of the therapeutic device to the top surface of the first layer of the porous dressing.

In one embodiment, a wound closure system may include a second layer of a porous dressing (e.g., a second mesh; a top mesh) having a top surface and a bottom surface. In one embodiment, the bottom surface of the second layer of the porous dressing is positioned over the therapeutic device (e.g., a drain; a suction device; a sensor) and the top surface of the first layer of the porous dressing. The second layer of the porous dressing may be flexible for conforming to the shape of the therapeutic device. In one embodiment, a bottom surface of the second layer of the porous dressing may be tacky and/or include an adhesive for securing the second layer of the porous mesh to one or more surfaces of the therapeutic device and/or the first layer of the porous dressing.

In one embodiment, one or more surfaces of the therapeutic device may be tacky and/or have an adhesive applied thereto for attaching the second layer of the porous dressing to the therapeutic device.

In one embodiment, with the second layer of the porous dressing positioned over the first layer of the porous dressing, and with the therapeutic device disposed therebetween, a flowable adhesive may be applied over the second layer of the porous dressing, the first layer of the porous dressing, and the therapeutic device that is disposed between the second layer of the porous dressing and the first layer of the porous dressing.

In one embodiment, the therapeutic device may be a suction device that is placed on top of a wound or incision and held in place by one or more layers of porous dressings and a topical skin adhesive. In one embodiment, the therapeutic device has one or more connection ports where fluid may be infused into and/or drained from a wound or incision. The one or more connection ports may be on a top or a side of the therapeutic device. In one embodiment, a first connection port may be attached to a vacuum via tubing for applying negative pressure to a wound or incision. In one embodiment, a second connection port may be attached to tubing that is configured for infusing a therapeutic solution (e.g., saline; medication) into a wound or incision.

In one embodiment, the therapeutic device may be transparent for enabling medical personnel to monitor the wound or incision during healing.

In one embodiment, the therapeutic device may be cut to a length that matches the length of the wound or incision.

In one embodiment, the therapeutic device may be attached to a pump or a drain.

In one embodiment, the therapeutic device may have a low profile to minimize the distance that the therapeutic device projects above a skin surface.

In one embodiment, the therapeutic device may be made of silicone.

The wound closure systems disclosed herein may be used on patients having significant comorbidities that require advanced wound closure approaches including providing appropriate skin tissue approximation/closure and negative pressure wound therapy to minimize post-surgical complications and delays during recovery.

The wound closure systems disclosed herein preferably enable medical personnel to achieve surgical wound approximation, wound closure, controlled removal of exudates, maintenance of closure over a healing period, not requiring dressing changes, and to apply negative pressure wound therapy in a controlled manner.

The wound closure systems disclosed may be used for wound drainage, wound irrigation, wound cleansing, antimicrobial irrigation, pain medication administration, etc.

In one embodiment, the wound closure system may be placed on top of a wound or incision and held in place by a topical skin adhesive. In one embodiment, the therapeutic device has one or more connection ports where fluid may be infused into and/or withdrawn from the wound or incision. The connection ports may be located on top or on the sides of the therapeutic device. One connection port may be attached to a vacuum via tubing for applying negative pressure to a wound or incision. One connection port may be attached to tubing for infusing saline or medication into a wound or incision.

In one embodiment not recited in the appended claims, a method of closing a wound may include approximating the edges of a wound or incision using a first layer of a porous dressing and positioning a therapeutic device (e.g., a drain; a suction device; a sensor) over the top surface of the first layer of the porous dressing.

In one embodiment not recited in the appended claims, the method preferably includes securing the therapeutic device over the top surface of the first layer of the porous dressing so that an elongated channel of the therapeutic device is positioned over the top surface of the first layer of the porous dressing with the elongated channel positioned adjacent the wound or incision.

In one embodiment not recited in the appended claims, the method preferably includes applying a second layer of a porous dressing over both the first layer of the porous dressing and the therapeutic device to secure the elongated channel of the therapeutic device to the top surface of the first porous dressing.

In one embodiment not recited in the appended claims, the method includes applying a flowable adhesive over the first layer of the porous dressing, the second layer of the porous dressing and the therapeutic device. The flowable adhesive may be cured for forming an air-tight seal around the wound or incision.

In one embodiment not recited in the appended claims, the method preferably includes attaching a tube to the therapeutic device for draining fluid (e.g., exudate) from the elongated channel of the therapeutic device or for infusing fluid into the elongated channel of the therapeutic device. In one embodiment, the tube may be attached to a vacuum for applying negative pressure to the wound for draining the wound. In one embodiment, the tube may be attached to a source of a therapeutic solution (e.g., saline; medication; an antibiotic solution) for infusing the therapeutic solution into the wound or incision.

In one embodiment, the elongated channel of the therapeutic device (e.g., a drain; a suction device) desirably has two connection ports where fluid may be infused into and/or withdrawn from the wound or incision.

In one embodiment, the connection ports may be on top or on one or more sides of the therapeutic device.

In one embodiment, a connection port may be attached to a vacuum via tubing, which is configured to apply negative pressure to a wound or incision.

In one embodiment, a connection port may be attached to an infusing tube for infusing a therapeutic solution (e.g., saline; medication; antibiotic) into a wound or incision.

In one embodiment, the elongated channel of a therapeutic device may have a T-shaped cross-sectional shape.

In one embodiment, the flowable adhesive cures to a transparent covering for enabling medical personnel to view the wound or incision during healing.

In one embodiment, the flowable adhesive is a silicone material.

In one embodiment, the therapeutic device preferably has a low profile with tapered edges (e.g., a speed bump shape).

In one embodiment, the therapeutic device has an elongated channel that is open at the bottom surface of the therapeutic device for providing suction that is directly in contact with the wound or incision.

These and other preferred embodiments of the present patent application will be described in more detail herein.

### BRIEF DESCRIPTION OF THE DRAWINGS

FIG. 1 is a schematic view of a wound closure system including a porous mesh having a top surface and a bottom surface, first pressure sensitive adhesive overlying the top surface of the porous mesh, second pressure sensitive adhesive overlying the bottom surface of the porous mesh, a removable top liner covering the top surface of the porous mesh, and a removable bottom liner covering the bottom surface of the porous mesh, in accordance with one embodiment of the present patent application.
FIG. 2 is a top view of the removable top liner shown in FIG. 1, the removable top liner including a center section and first and second peripheral sections that bound the center section, in accordance with one embodiment of the present patent application.
FIG. 3 is a bottom view of the removable bottom liner shown in FIG. 1, the removable bottom liner including a center section and first and second peripheral sections that bound the center section, in accordance with one embodiment of the present patent application.
FIG. 4 is a top view of the porous mesh shown in FIG. 1, the top surface of the porous mesh being covered by the first pressure sensitive adhesive, in accordance with one embodiment of the present patent application.
FIG. 5 is a bottom view of the porous mesh shown in FIG.1, the bottom surface of the porous mesh being covered by the second pressure sensitive adhesive, in accordance with one embodiment of the present patent application.
FIG. 6 shows the bottom surface of the porous mesh of FIG. 1 secured to skin by the second pressure sensitive adhesive, and a therapeutic device secured over the top surface of the porous mesh by the first pressure sensitive adhesive, in accordance with one embodiment of the present patent application.
FIG. 7 is a top view of the porous mesh and the therapeutic device shown in FIG. 6.
FIG. 8 shows a method of applying a liquid adhesive over the porous mesh and the therapeutic device shown in FIGS. 6 and 7, in accordance with one embodiment of the present patent application.
FIG. 9A shows a first stage of a method of securing a wound closure system over an incision formed in skin of a patient, in accordance with one embodiment of the present patent application.
FIG. 9B shows a second stage of a method of securing a wound closure system over the incision formed in skin of the patient, in accordance with one embodiment of the present patent application.
FIG. 9C shows a third stage of a method of securing a wound closure system over the incision formed in skin of the patient, in accordance with one embodiment of the present patent application.
FIG. 9D shows a fourth stage of a method of securing a wound closure system over the incision formed in skin of the patient, in accordance with one embodiment of the present patent application.
FIG. 9E shows a fifth stage a method of securing a wound closure system over the incision formed in skin of the patient, in accordance with one embodiment of the present patent application.
FIG. 10A shows a porous mesh being secured over an incision formed in a patient, in accordance with one embodiment of the present patent application.
FIG. 10B shows a releasable liner being peeled away from a top surface of the porous mesh shown in FIGS. 10A, in accordance with one embodiment of the present patent application.
FIG. 10C shows an elongated tube having mesh wings being secured over the top surface of the porous mesh shown in FIG. 10B, in accordance with one embodiment of the present patent application.
FIG. 10D shows a liquid adhesive being applied over both the elongated tube having mesh wings and the porous mesh shown in FIGS. 10B and 10C, in accordance with one embodiment of the present patent application.
FIG. 11A shows a porous mesh being secured over an incision formed in a patient, the porous mesh having first and second peripheral sections of a releasable top liner overlying a top surface of the porous mesh, in accordance with one embodiment of the present patent application.
FIG. 11B shows the porous mesh of FIG. 11A after the first and second peripheral sections of the releasable top liner have been peeled away to fully expose the top surface of the porous mesh, in accordance with one embodiment of the present patent application.
FIG. 11C shows an elongated tube positioned over the top surface of the porous mesh shown in FIG. 11B, the elongated tube being in general alignment with the incision formed in the patient, in accordance with one embodiment of the present patent application.
FIG. 11D shows a curable adhesive being applied over both the elongated tube and the top surface of the porous mesh shown in FIG. 11C, in accordance with one embodiment of the present patent application.
FIG. 12 shows a perspective view of a wound closure system including a porous mesh approximating skin edges of the incision shown in FIG. 11B, the elongated tube of FIG. 11D secured over the incision, a drain tube in fluid communication with the elongated tube for draining bodily fluids from the incision, and an injection tube in fluid communication with the elongated tube for infusing saline or medication over the incision shown, in accordance with one embodiment of the present patent application.
FIG. 13 is a top plan view of a porous mesh having a releasable liner that covers a central region of a top surface of the porous mesh, in accordance with one embodiment of the present patent application.
FIG. 14A shows the porous mesh with the releasable liner of FIG. 13 positioned over a wound of a patient, in accordance with one embodiment of the present patent application.
FIG. 14B shows a liquid adhesive applied over the porous mesh and the releasable liner shown in FIG. 14A.
FIG. 14C shows the porous mesh and the liquid adhesive of FIG. 14B after the releasable liner of FIG. 14B has been peeled away from the top surface of the porous mesh to expose the central region of the top surface of the porous mesh.
FIG. 14D shows a therapeutic device positioned over the central region of the top surface of the porous mesh shown in FIG. 14C.
FIG. 15 shows a cross-sectional view of a therapeutic device including elongated channels that extend along a length of the therapeutic device and fenestrations that are in communication with the elongated channels, in accordance with one embodiment of the present patent application.

### DETAILED DESCRIPTION OF PREFERRED EMBODIMENTS

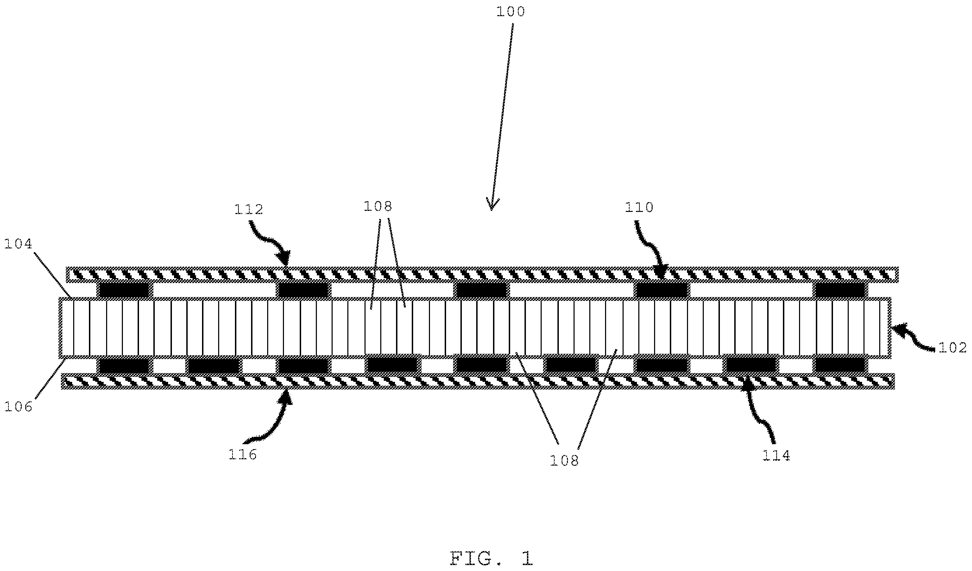

Referring to FIG. 1, in one embodiment, a wound closure system 100 includes a porous mesh 102 having a top surface 104, a bottom surface 106 and a plurality of openings 108 (e.g., pores) that extend between the top and bottom surfaces of the porous mesh. In one embodiment, the porous mesh may be used for approximating the opposing skin edges of a wound or incision. As used herein, the terms wound, and incision may be used interchangeably. As used herein, the terms wound approximation, incision approximation, and skin approximation may be used interchangeably.

The porous mesh 102 may be woven, non-woven, extruded, punched, perforated, molded, etc. In one embodiment, the porous mesh may be similar to a mesh patch sold under the federally registered trademark PRINEO^{®} by Ethicon, Inc. of Somerville, New Jersey. In one embodiment, the porous mesh 102 may be coated and/or impregnated with an initiator or accelerator of polymerization.

The porous mesh 102 may be of any shape that is necessary to cover a wound or incision, such as elliptical, circular, rectangular, square, etc. The porous mesh may have a length to width ratio of about 2:1 to 20:1. In one embodiment, the length of the porous mesh 102 may be about 10-50 cm. In one embodiment, the width of the porous mesh 102 may be about 2-10 cm.

In one embodiment, the openings 108 of the porous mesh 102 may be of any shape, including round, rectangular, triangular, elliptical, etc. The openings 108 may have diameters of about 0.2 - 2 mm. In one embodiment, the areas of the individual openings 108 may be from about 0.04 mm² to about 4 mm².

The wound closure system 100 includes a first pressure sensitive adhesive 110 that overlies the top surface 104 of the porous mesh 102. A removable top liner 1 12 covers the first pressure sensitive adhesive 110 and the top surface 104 of the porous mesh 102. In one embodiment, the removable top liner may be peeled away from the top surface 104 of the porous mesh 102 for exposing the first pressure sensitive adhesive 110 that overlies the top surface of the porous mesh. The first pressure sensitive adhesive 110 that overlies the top surface 104 of the porous mesh 102 may be utilized for securing a therapeutic device, such as a negative pressure wound therapy (NPWT) device, over the top surface of the porous mesh.

The wound closure system 100 includes second pressure sensitive adhesive 114 that overlies the bottom surface 106 of the porous mesh. A removable bottom liner 116 desirably covers the second pressure sensitive adhesive 114 and the bottom surface 106 of the porous mesh 102. The removable bottom liner may be peeled away from the bottom surface 106 of the porous mesh 102 for exposing the second pressure sensitive adhesive 114 that overlies the bottom surface of the porous mesh. The second pressure sensitive adhesive 114 that overlies the bottom surface 106 of the porous mesh 102 may be utilized for securing the porous mesh to skin of a patient for approximating the opposing edges of a wound or incision.

In one embodiment, the releasable top and bottom liners 112, 116 may be made of synthetic or natural polymers that are easily peelable from the respective first and second pressure sensitive adhesives 110, 114.

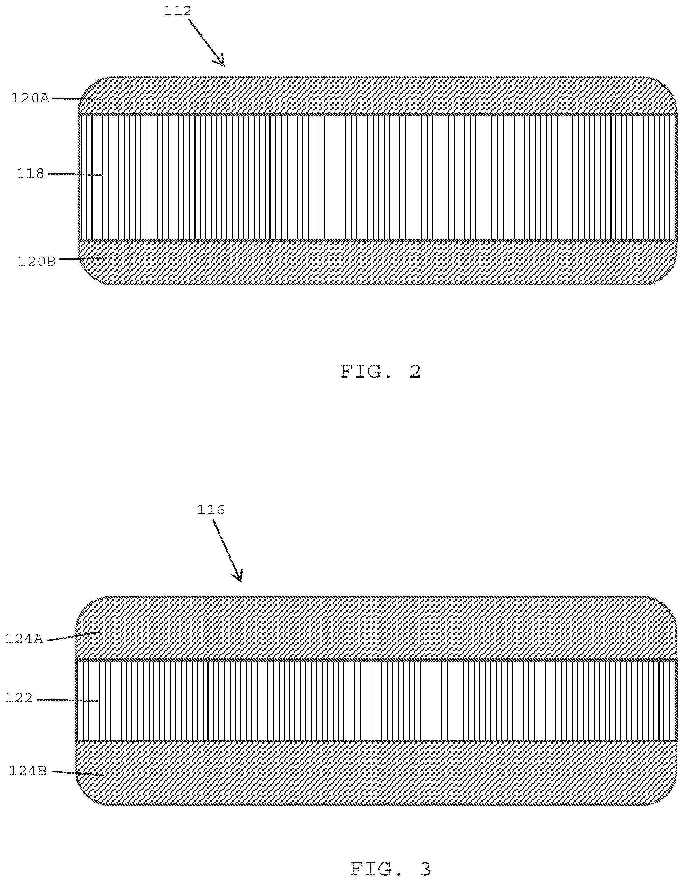

Referring to FIG. 2, in one embodiment, the removable top liner 112 that covers the top surface 104 of the porous mesh 102 (FIG. 1) preferably includes a center segment 118 that extends over a center of the porous mesh and first and second peripheral segments 120A, 120B that bound opposite lateral sides of the center segment 118. In one embodiment, the center segment 118 and the first and second peripheral segments 120A, 120B of the removable top liner 112 may be peeled away from the top surface 104 of the porous mesh 102 (FIG. 1) for exposing the first pressure sensitive adhesive and the top surface of the porous mesh. The center segment 118 and the first and second peripheral segments may be removed independently of one another from the top surface of the porous mesh. For example, the center segment 118 of the removable top liner 112 may be peeled away from the porous mesh so that a surgeon can view a wound or an incision through the porous mesh for aligning the porous mesh with the incision. When aligning the porous mesh with the wound or incision, the first and second peripheral segments may be grasped by a surgeon for controlling and manipulating the porous mesh. In one embodiment, after the porous mesh has been secured to skin, the first and second peripheral segments 120A, 120B of the removable top liner 112 may be peeled away from the top surface of the porous mesh.

Referring to FIG. 3, in one embodiment, the removal bottom liner 116 desirably covers the bottom surface 106 of the porous mesh 102 (FIG. 1). In one embodiment, the removable bottom liner 116 preferably includes a center segment 122 and first and second peripheral segments 124A, 124B that bound opposite lateral sides of the center segment 122. In one embodiment, the center segment 122 and the first and second peripheral segments 124A, 124B of the removable bottom liner 116 may be peeled away from the bottom surface 106 of the porous mesh 102 (FIG. 1) independently of one another. For example, the center segment 122 of the removable bottom liner 116 may be peeled away from the bottom surface of the porous mesh so that a surgeon can view an incision through the porous mesh for aligning the porous mesh with the incision, whereupon the surgeon may approximate the opposing edges of the incision. The first and second peripheral segments 120A, 120B may initially remain in place as the bottom surface of the porous mesh is used for approximating skin edges. In one embodiment, after the porous mesh has been properly aligned with the incision, and after the opposing edges of the incision have been approximated, the first and second peripheral segments 120A, 120B of the removable bottom liner 116 may be peeled away from the bottom surface of the porous mesh for further securing the bottom surface of the porous mesh to skin that surrounds the incision.

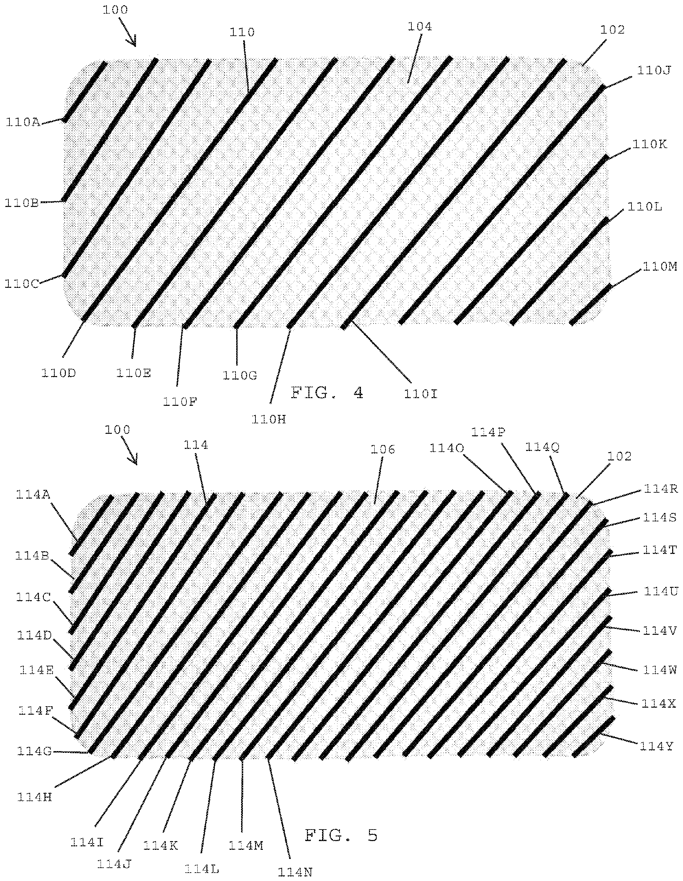

Referring to FIG. 4, the wound closure system 100 includes the first pressure sensitive adhesive 110 (FIG. 1) that overlies the top surface 104 of the porous mesh 102. In one embodiment, the first pressure sensitive adhesive 110 may include a plurality of first pressure sensitive adhesive traces 110A-110M that extend over the top surface 104 of the porous mesh 102. The first pressure sensitive adhesive traces 110A-110M may be spaced from one another over the top surface of the porous mesh.

The first pressure sensitive adhesive traces 110A-110M may be in a form of linear segments of pressure sensitive adhesive (as shown in FIG. 4) and may extend at any angle relative to the length of the porous mesh 102. In one embodiment, the first pressure sensitive adhesive 110 overlying the top surface 104 of the porous mesh 102 may have a non-linear shape such as round, square, elliptical, dots, etc.

Referring to FIG. 5, the wound closure system 100 includes the second pressure sensitive adhesive 114 (FIG. 1) that overlies the bottom surface 106 of the porous mesh 102. In one embodiment, the second pressure sensitive adhesive 114 may include a plurality of second pressure sensitive adhesive traces 114A-114Y that extend over the bottom surface 106 of the porous mesh 102. The second pressure sensitive adhesive traces 114A-110Y may be spaced from one another over the bottom surface of the porous mesh.

The second pressure sensitive adhesive traces 114A-114Y may be in a form of linear segments of pressure sensitive adhesive (as shown in FIG. 5) and may extend at any angle relative to the length of the porous mesh 102. In one embodiment, the second pressure sensitive adhesive 114 over the bottom surface 106 of the porous mesh 102 may have a non-linear shape such as round, square, elliptical, dots, etc.

Referring to FIGS. 4 and 5, in one embodiment, the number of the second pressure sensitive adhesive traces 114A-114Y that cover the bottom surface 106 of the porous mesh 102 is greater than the number of the first pressure sensitive adhesive traces 110A-110M that cover the top surface 104 of the porous mesh. In one embodiment, the first pressure sensitive adhesive traces 110A-110M that cover the top surface of the porous mesh 102 may comprise an adhesive that is weaker than the adhesive used for forming the second pressure sensitive adhesive traces 114A-114Y that cover the bottom surface of the porous mesh. In one embodiment, the density of the first pressure sensitive adhesive traces 110A-110M that cover the top surface of the porous mesh 102 may be less than the density of the second pressure sensitive adhesive traces 114A-114Y that cover the bottom surface of the porous mesh. Utilizing pressure sensitive adhesive traces that are weaker and/or less dense over the top surface of the porous mesh will ensure that removal of the top liner 112 will not pull the porous mesh 102 off of the skin, that the removal of the top liner will not change skin edge approximation, and that a therapeutic device may be lifted away from and repositioned over the top surface of the porous mesh without pulling the porous mesh away from the patient's skin.

In one embodiment, the first and second pressure sensitive adhesives may comprise water soluble pressure sensitive adhesives including hydrocolloids; homo-polymer emulsion (PVA); water-based acrylic adhesives; polyurethane dispersions (PUDs); polyethylene glycol; dextrin/starch-based adhesives; N-vinyl pyrrolidone copolymers; polyvinyl alcohol; cellulose ethers; methylcellulose; carboxymethylcellulose; polyvinylpyrrolidone; polyvinyl acetates, or water insoluble pressure sensitive adhesives including acrylic adhesives; cyanoacrylate adhesives; epoxy; silicone based adhesives; and urethane.

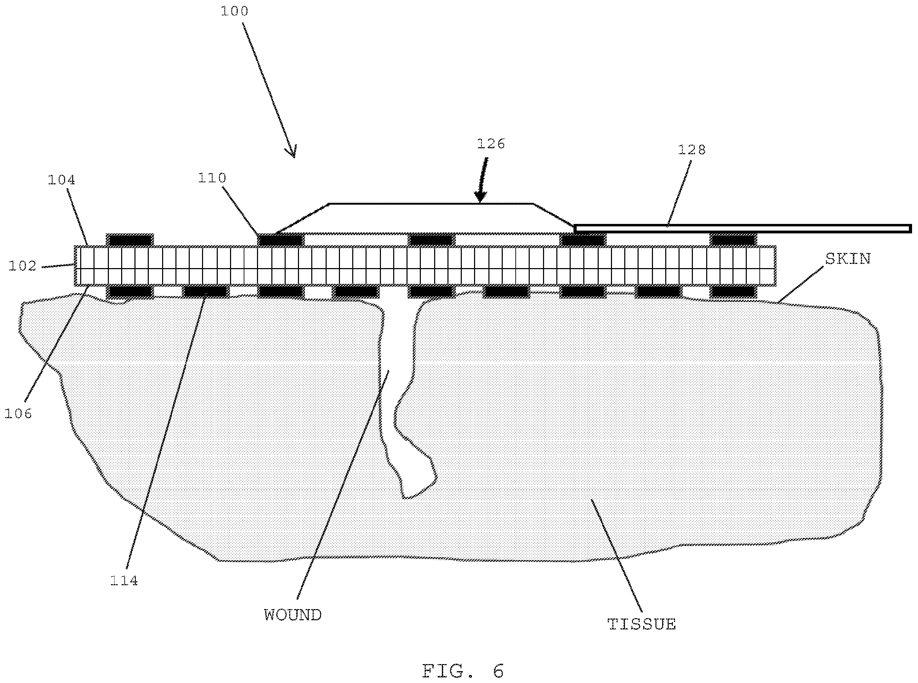

Referring to FIG. 6, in one embodiment, the porous mesh 102 is positioned over a wound formed in tissue. The porous mesh is preferably utilized for approximating the opposing skin edges of the wound. The removable bottom liner 116 (FIG. 1) is peeled away from the bottom surface 106 of the porous mesh 102 to expose the second pressure sensitive adhesive 114 (e.g., traces 114A-114Y shown in FIG. 5) that overlies the bottom surface of the porous mesh. The second pressure sensitive adhesive 114 overlying the bottom surface of the porous mesh 102 may be pressed against the skin of a patient for securing the bottom surface 106 of the porous mesh 102 to the skin and/or for approximating the opposing skin edges of the wound.

The porous mesh 102 is used to approximate and hold in apposition or close approximation the edges of the wound, using the second pressure sensitive adhesive traces 114A-114Y (FIG. 5) for securing the porous mesh and for securing in close approximation or apposition the opposing edges of the wound.

In one embodiment, after the porous mesh 102 has been secured to the skin for approximating the skin edges of the wound, the removable top liner 112 (FIG. 1) may be peeled away from the top surface 104 of the porous mesh 102 to expose the first pressure sensitive adhesive 110 that overlies the top surface 104 of the porous mesh 102.

In one embodiment, a therapeutic device 126, such as a negative pressure wound therapy (NPWT) device, may be pressed onto the first pressure sensitive adhesive 110 that overlies the top surface 104 of the porous mesh 102 for securing the therapeutic device 126 over the top surface 104 of the porous mesh 102. The therapeutic device may be activated for generating negative pressure and/or a vacuum for draining bodily fluids from the wound. In one embodiment, the wound closure system 100 preferably includes a cannula 128 that may be coupled with the therapeutic device 126 for draining fluids (e.g., wound exudate) that are suctioned by the therapeutic device 126.

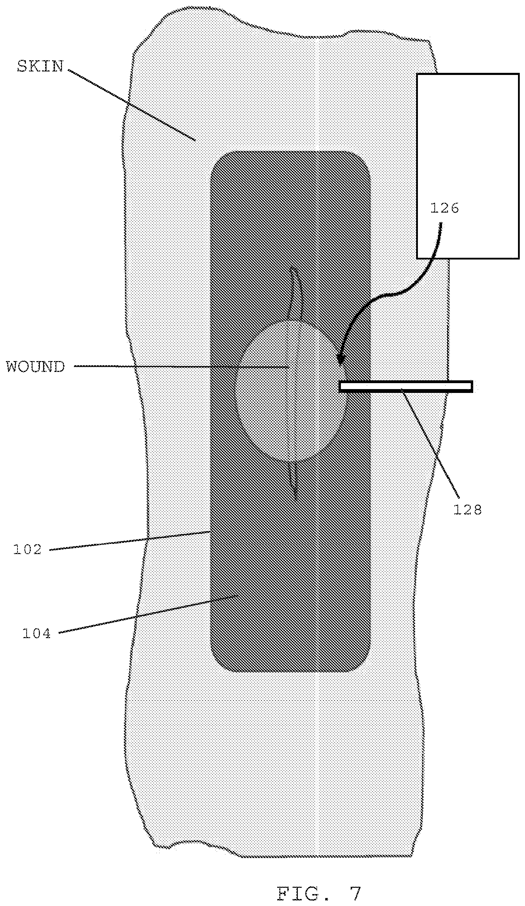

Referring to FIG. 7, in one embodiment, the porous mesh 102 is secured over the wound. The second pressure sensitive adhesive 114 (FIG. 6) overlying the bottom surface of the porous mesh is utilized for securing the bottom surface of the porous mesh 102 to the skin. In one embodiment, the wound is visible through the porous mesh 102, which facilitates alignment of the porous mesh with the wound. A surgeon may manipulate the porous mesh 102 (e.g., repeatedly lifting, repositioning and/or smoothing the porous mesh) for approximating the opposing skin edges of the wound. In one embodiment, the therapeutic device 126 may be pressed against the first pressure sensitive adhesive 110 (FIG. 6) that overlies the top surface 104 of the porous mesh 102 for adhering the therapeutic device 126 to the top surface of the porous mesh. The canula 128 may be connected with the therapeutic device 126 for draining fluid away from the wound.

In one embodiment, the longitudinal axis of the porous mesh 102 is aligned with and superimposed over the wound. The positioning of porous mesh over the wound is performed so that the longitudinal axis is aligned with the wound and overlaps the ends of the wound (i.e., the longitudinal axis of the porous mesh is in registration the wound or surgical incision).

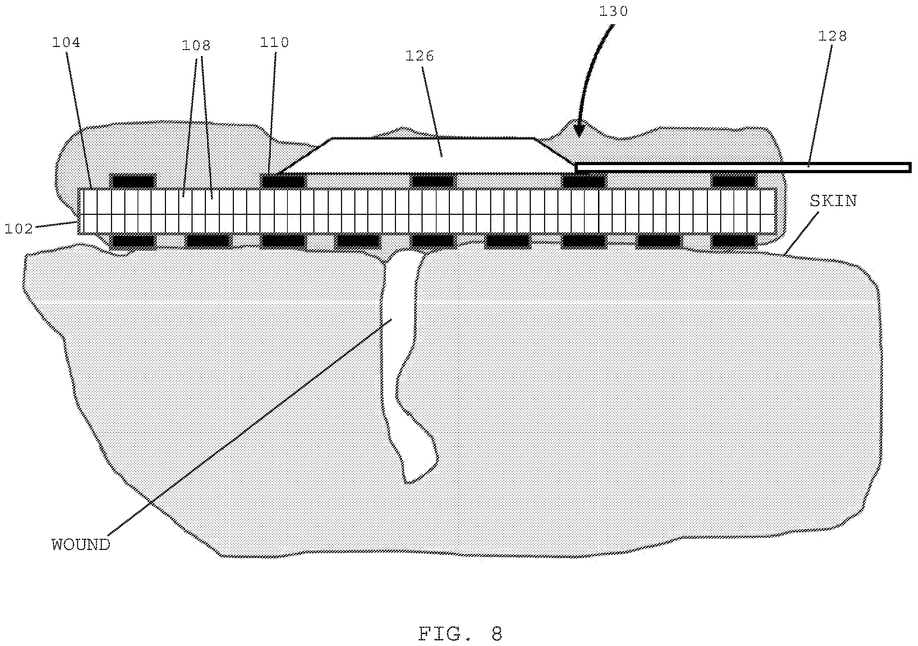

Referring to FIG. 8, a liquid adhesive 130 (e.g., a rapidly polymerizable or cross-linkable liquid adhesive) is applied over the therapeutic device 126 and the porous mesh 102 that is secured to skin for approximating skin edges of the wound. In one embodiment, before curing, the liquid adhesive 130 may penetrate through the openings 108 of the porous mesh 102 for establishing contact with skin 100 in all areas not covered by the therapeutic device 126. In one embodiment, after curing, the liquid adhesive 130 preferably forms an air-tight seal around the therapeutic device 126 and the periphery of the porous mesh 102.

The liquid adhesive 130 may be a polymerizable or cross-linkable adhesive that is applied over the porous mesh 102 and the therapeutic device 126. The liquid adhesive 130 may be expressed from a container having a porous tip impregnated with a polymerization or crosslinking accelerator or initiator.

In one embodiment, the liquid adhesive does not contain a polymerization or crosslinking accelerator or initiator, rather the polymerization or cross-linking accelerator or activator/initiator may be present on or in the porous mesh 102 in a releasable or reactive form (i.e., available for rapid reaction when contacted by the liquid adhesive 130).

In one embodiment, after being applied over the porous mesh 102 and the therapeutic device 126, the liquid adhesive 130 preferably polymerizes and/or cross-links and solidifies, establishing a secure bond with the skin, the porous mesh 102, and the therapeutic device 126.

In one embodiment, the liquid adhesive 130 is polymerized or is crosslinked polymerized or is cross-linked after coming in contact with initiators and/or accelerators of adhesive polymerization and/or cross-linking, including naturally found initiators on the tissue, such as moisture, traces of proteins, etc. Such initiators and/or accelerators may be coated or disposed non-releasably, i.e., immobilized in or on the porous mesh 102 while retaining activity to initiate or accelerate polymerization and/or cross-linking. In one embodiment, initiators and/or accelerators are disposed releasably, i.e., they can be at least partially released into and mix with the liquid adhesive 130.

In one embodiment, the liquid adhesive 130 is polymerized or is cross-linked after coming in contact with initiators and/or accelerators releasably disposed in or on the porous mesh 102. Rapid polymerization and/or crosslinking of the liquid adhesive 130 results in bonding the porous mesh 102 to tissue (e.g., skin).

The liquid adhesive 130 may be any type of biocompatible and rapidly cross-linkable and/or polymerizable compound or mixture of compounds. As used herein, the terms rapidly cross-linkable and/or polymerizable mean that after initiators or accelerators are added, or after the adhesive is formed from two or more components, it is capable of curing, i.e., cross-linking and/or polymerizing within 0.2 minutes to about 20 minutes, and more preferably within 0.5-10 minutes. In one embodiment, the liquid adhesive may cure in about one minute.

In one embodiment, the liquid adhesive 130 may be formed prior to application onto the porous mesh 102, for instance by mixing two components contained in separate barrels or a two-barrel syringe, and passing the two components through a mixing tip. In this embodiment, there may be no crosslinking initiator or accelerator disposed inside of porous mesh 20. In one embodiment, the liquid adhesive 130 may be formed by mixing fibrinogen and thrombin together.

In one embodiment, the liquid adhesive 130 may comprise fibrinogen, and a crosslinking initiator or accelerator that is disposed inside the porous mesh 102 comprises thrombin.

Many other adhesive formulations may be used and are known to a skilled artisan. For example, mixtures containing PEG succinimidyl glutarate may be used as a liquid adhesive.

In certain embodiments, initiators and/or accelerators or rate modifiers of adhesive polymerization or cross-linking may be releasably disposed on the porous mesh 102 or releasably incorporated into the porous mesh. For example, one or more chemical substances may be dispersed in or on the porous mesh such as being chemically bound, physically bound, coated, absorbed, or adsorbed to the porous mesh.

For example, a polymerization initiator or accelerator or rate modifier may be loaded in or on the porous mesh so that the initiator or rate modifier provides the desired initiation or rate modification effect to a subsequently applied polymerizable adhesive composition. The polymerization initiator or rate modifier may be immobilized in or on porous mesh so that the initiator or rate modifier does not become detached from the porous mesh and its residues are dispersed in the resultant polymeric material. Alternatively, for example, the polymerization initiator or rate modifier may be initially attached to the porous mesh, but only in such a manner that it becomes mobilized or solubilized by a subsequently applied polymerizable adhesive composition and dispersed in the resultant polymeric material.

If desired, a combination of chemical substances may also be provided in or on porous mesh, to provide multiple effects. For example, a first chemical species (such as a polymerization initiator or rate modifier) may be immobilized in or on the mesh, while a second, different chemical species (such as a bioactive material) may be detachably attached to the mesh.

When present in or on the porous mesh, the chemical substances (i.e., polymerization initiator, rate modifier, and/or bioactive materials, or other additives), may be incorporated in or on the porous mesh in any suitable manner. For example, the chemical substance may be added to the porous mesh by contacting the mesh with a solution, mixture, or the like including the chemical substances. The chemical substance may be added to the mesh, for example, by dipping, spraying, roll coating, gravure coating, brushing, vapor deposition, or the like. Alternatively, the chemical substance may be incorporated into or onto the porous mesh during manufacture of the mesh, such as during molding.

The polymerization initiator or rate modifier loaded in or on the porous mesh may provide a number of advantages (e.g., to provide shorter polymerization time). The concentration of the polymerization initiator or rate modifier may be increased to provide even shorter (i.e., faster) polymerization time. Because the polymerization initiator or rate modifier is loaded directly in or on the porous mesh, it is not necessary to mix the polymerizable adhesive composition with a polymerization initiator or rate modifier prior to application. This may allow a longer working time, where the polymerizable monomer composition may be more precisely and carefully applied over a longer period of time. Such suitable initiators are known in the art and are described, for example, in U.S. Patent Nos. 5,928,611 and 6,620,846 and U.S. Patent Application Publication No. 2002/0037310.

In one embodiment, the therapeutic device 126 is a negative pressure wound therapy (NPWT) device that is configured for generating negative pressure for draining exudate from the wound. The wound exudate drawn into the negative pressure wound therapy device 126 may be directed into the cannula 128 for draining the wound exudate away from the patient.

### Use of Wound Closure Systems.

In one embodiment, application of a wound closure system may be performed in one or more of the steps disclosed below.

In one embodiment, standard surgical practices are utilized for preparing the wound including thorough wound cleansing before application of the porous mesh 102 (FIG. 8). The standard surgical practices may include cleansing, irrigating, debriding, obtaining hemostasis, and closing deep tissue layers so that there is no tension on the wound edges. The wound edges are preferably closely approximated prior to application of the porous mesh so that significant manual approximation is not required during application of the porous mesh.

In one embodiment, a sterile gauze may be used to dry the wound, such as patting the wound with the sterile gauze. Drying the wound will ensure direct tissue contact for adherence of the second pressure sensitive adhesive 114 and the porous mesh 102 (FIG. 8) around the wound.

In one embodiment, the wound closure system including the porous mesh, the therapeutic device, and the liquid adhesive are preferably aseptically transferred into a sterile field.

Referring to FIGS. 1 and 3, in one embodiment, the center section 122 of the bottom liner 116 may be peeled away from the bottom surface 106 of the porous mesh 102.

In one embodiment, a surgeon will hold the mesh 102 by the corners of the peripheral sections 124A, 124B of the bottom liner 116 (FIG. 3) for ensuring the second pressure sensitive adhesive 114 (FIG. 8) applied to the bottom surface 106 of the porous mesh 102 may be adhered to the patient's skin.

In one embodiment, the porous mesh is positioned so that ½ of the mesh is on either side of the wound or incision, ensuring approximately 1 cm of the mesh extends beyond the ends of the wound or incision. In one embodiment, a surgeon gently presses on the porous mesh to ensure intimate contact of the bottom surface of the porous mesh to a selected side of the wound. In one embodiment, the porous mesh may be gently pulled perpendicularly over the wound while adjusting the porous mesh with fingers or forceps to achieve skin edge approximation and affix the remainder of the porous mesh to the other side of the wound. If there are areas where the porous mesh is loose, a surgeon may gently pass a gloved finger or instrument over the affected area of the porous mesh to ensure complete adherence of the mesh to the skin.

In one embodiment, after the skin edges have been approximated and the porous mesh is deemed to be properly affixed to the skin, the first and second peripheral sections 124A, 124B of the removable bottom liner 116 may be peeled away from the bottom surface of the porous mesh for adhering peripheral sections of the porous mesh to the skin.

In one embodiment, a sterile cutting tool may be utilized to trim the ends of the porous mesh, if necessary, to ensure that about one (1) cm of porous mesh material extends beyond the ends of the wound or incision.

In one embodiment, the first and second peripheral sections of the removable top liner 112 (FIG. 2) may be peeled away from the top surface of the porous mesh.

In one embodiment, the therapeutic device 126 may be pressed against the first pressure sensitive adhesive 110 applied to the top surface 104 of the porous mesh 102 to secure the therapeutic device in place atop the porous mesh.

In one embodiment, after medical personnel ensure that the porous mesh is in intimate contact with the skin and after the therapeutic device is secured atop the porous mesh, the liquid adhesive may be applied.

In one embodiment, immediately after the therapeutic device has been placed atop the porous mesh, the liquid adhesive 130 is preferably applied over the porous mesh and at least a portion of the therapeutic device 126. In one embodiment, immediately prior to applying and spreading the liquid adhesive, in the event of bodily fluid seepage, medical personnel may use a sterile gauze to dry the deployed porous mesh.

Referring to FIG. 8, in one embodiment, the liquid adhesive 130 is spready smoothly and evenly over the therapeutic device 126, at least a portion of the cannula 128, and the entire length of the porous mesh 102 and the surrounding skin area using a tool such as a spreader or a flexible applicator tip. The liquid adhesive is preferably spread using short strokes and moving from one end of the mesh to the other end, making sure that the therapeutic device is covered, and the porous mesh is saturated as the liquid adhesive is applied along the entire length of the porous mesh. The liquid adhesive 130 may also be applied over the edges or outer periphery of the porous mesh, covering a small margin of surrounding skin.

Once the liquid adhesive is applied to over the porous mesh and the therapeutic device, after about 1 minute, medical personnel may check that polymerization is complete by gently dabbing along the length of the porous mesh with a gloved finger, checking for tackiness. When no liquid or tackiness is apparent, the polymerization process is complete. Once the liquid adhesive is polymerized, the therapeutic device may be operated for performing various therapeutic activities including but not limited to draining bodily fluids from the wound, infusing the wound with saline or medications, and/or monitoring the wound using a sensor.

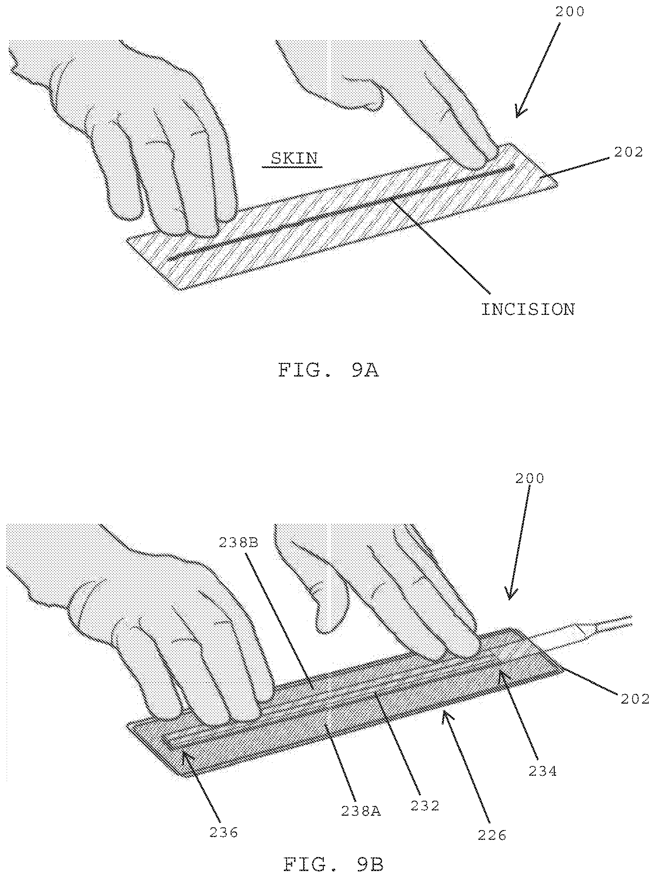

Referring to FIG. 9A, in one embodiment, after an incision (e.g., a wound) is formed in skin of a patient, a wound closure system 200 may be applied over the incision for approximating skin edges and promoting healing of the wound. In one embodiment, the wound closure system 200 preferably includes a porous mesh 202 that is adhered to skin that surrounds the incision. The porous mesh 202 may be at least partially transparent so that a surgeon may view the incision through the porous mesh for effectively aligning the porous mesh with the incision and for enabling the physician to approximate the opposing skin edges of the incision. The porous mesh preferably has a length that is greater than the length of the incision. In one embodiment, the porous mesh may be repeatedly lifted away from the skin for repositioning the porous mesh to ensure that the porous mesh is properly aligned with the incision and/or that the opposing skin edges of the incision are approximated.

Referring to FIG. 9B, in one embodiment, the wound closure system 200 preferably includes a therapeutic device 226 that may be positioned over a top surface of the porous mesh 202. In one embodiment, the therapeutic device 226 preferably includes an elongated tube 232 having a proximal end 234 and a distal end 236. The elongated tube 232 desirably has a length that extends from the proximal end 234 to the distal end 236 thereof. In one embodiment, the therapeutic device 226 preferably includes first and second mesh wings 238A, 238B that extend along opposite lateral sides of the elongated tube 232. In one embodiment, the first and second mesh wings 238A, 238B completely surround the sides of the elongated tube 232 and extend beyond the respective proximal and distal ends 234, 236 of the elongated tube 232. The outer perimeter of the first and second mesh wings 238A, 238B may be smaller than the outer perimeter of the porous mesh 202 so that when the therapeutic device 226 is secured atop the porous mesh 202 (FIG. 9A), the elongated tube 232 and the first and second mesh wings 238A, 238B are located entirely within the outer perimeter of the porous mesh.

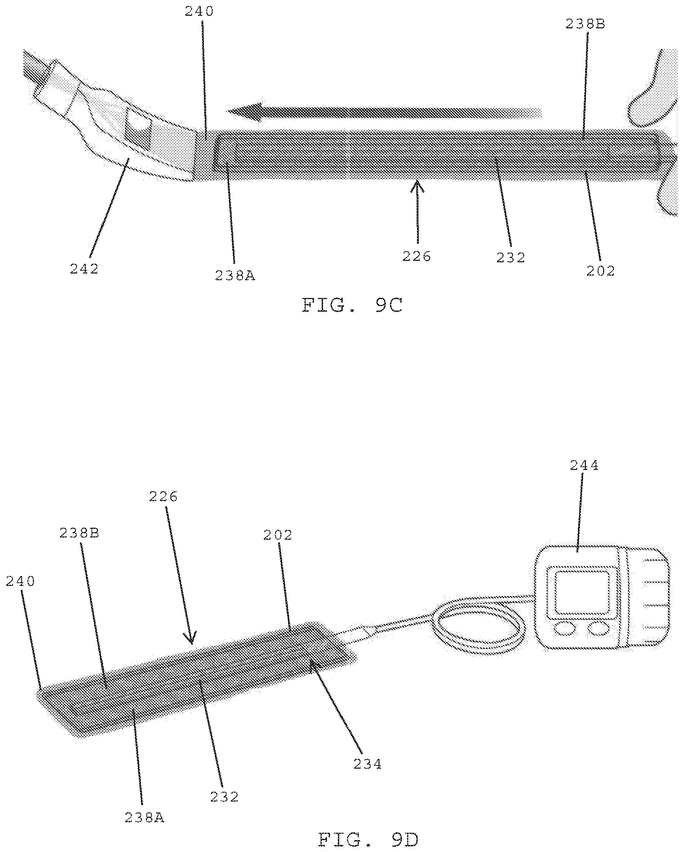

Referring to FIG. 9C, in one embodiment, after the elongated tube 232 and the first and second mesh wings 238A, 238B of the therapeutic device 226 have been secured over a top surface of the porous mesh 202, a liquid adhesive 240 may be spread over the top of the elongated tube 232, the first and second mesh wings 238A, 238B and the porous mesh 202. The liquid adhesive 240 may be spread using a topical adhesive spreader 242 (e.g., a tool having a flexible blade; a brush). The liquid adhesive 240 preferably completely surrounds the outer perimeter of the porous mesh 202 and completely encapsulates the therapeutic device 226 and the porous mesh 202.

In one embodiment, the first and second mesh wings 238A, 238B are preferably utilized for positioning the elongated tube 232 over the incision. In one embodiment, the elongated tube 232 and the first and second mesh wings 238A, 238B may be cut to the length of the incision.

Referring to FIG. 9D, after the liquid adhesive 240 has been spread over the elongated tube 232, the first and second mesh wings 238A, 238B and the porous mesh 202, a wound treatment controller 244 may be coupled with the proximal end 234 of the elongated tube 232 of the therapeutic device 226. In one embodiment, the wound treatment controller 244 is configured for draining fluid from the incision/wound and/or infusing saline and/or medication over the incision/wound for promoting healing.

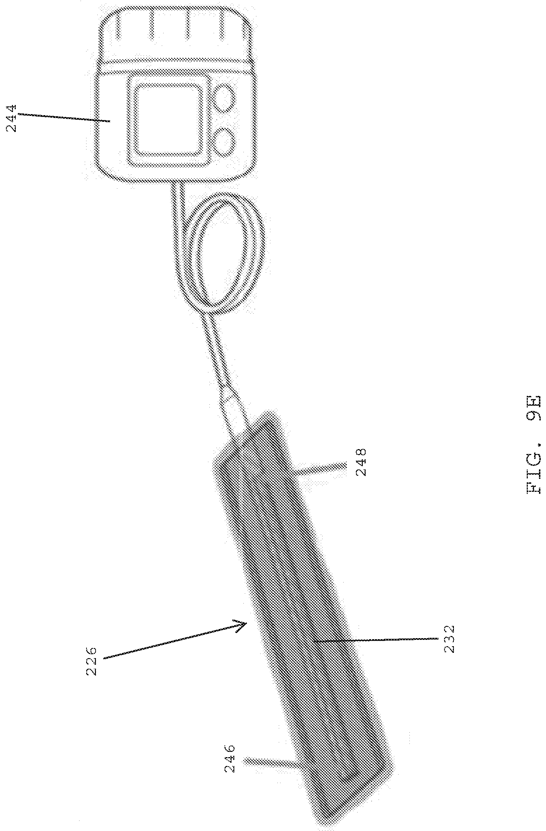

Referring to FIG. 9E, in one embodiment, the wound treatment controller 244 is preferably coupled with the proximal end 234 of the elongated tube 232 of the therapeutic device 226. In one embodiment, the elongated tube 232 has a first inlet 246 that is preferably configured for draining fluids (e.g., wound exudate) from the incision (FIG. 9A) and a second inlet 248 that is preferably configured for delivering saline or medication to the incision (FIG. 9A).

In one embodiment, the elongated tube 232 may have holes and/or slits that are used for draining fluid from a wound/incision or infusing saline or a medication onto the wound/incision. In one embodiment, the elongated tube 232 may be placed on top of a porous mesh and may be adhered to the porous mesh by using a pressure sensitive adhesive.

In one embodiment, the elongated tube 232 may have two ends, with one end of the elongated tube being attached to a vacuum for draining fluids (e.g., wound exudate) from the wound/incision and one end of tubing being attached to an inlet port for infusing/injecting fluids, saline irrigation, or medication over the wound/incision. The elongated tube is customizable. The length of the elongated tube may be adjusted, and different therapeutic devices may be used with the elongated tube to monitor the incision, etc. The elongated tube may also be flexed (e.g., bent into a curved configuration) for conforming to the shape of the wound/incision.

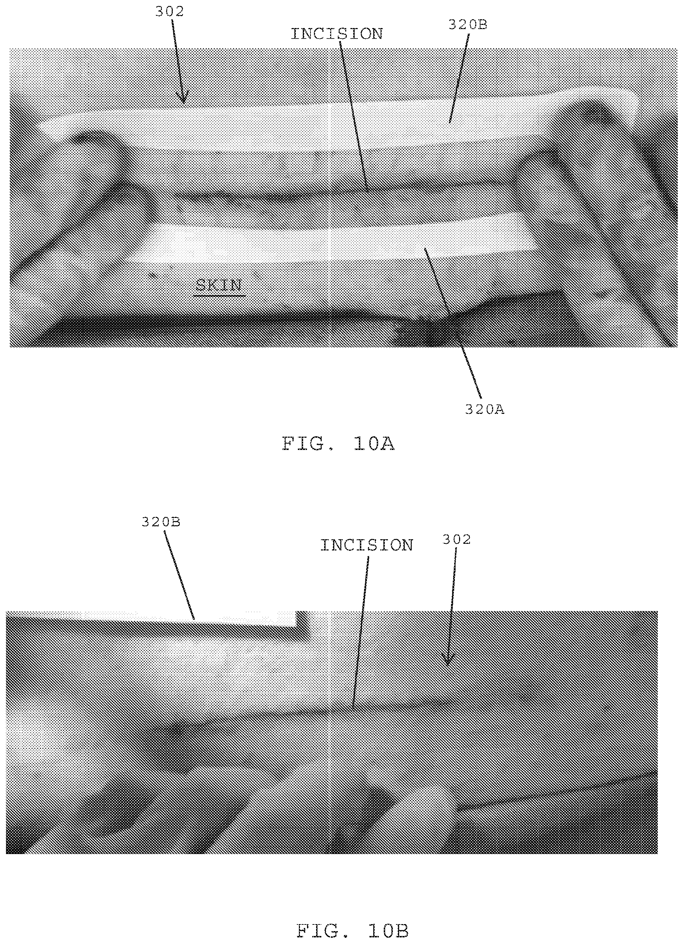

Referring to FIG. 10A, in one embodiment, a porous mesh 302 is preferably positioned over an incision formed in skin of a patient. A bottom surface of the porous mesh 302 may have a pressure sensitive adhesive for adhering the bottom surface of the porous mesh to the skin of the patient. When the porous mesh 302 has been positioned over the incision, the porous mesh is preferably aligned with the incision and preferably approximates the opposing skin edges of the incision.

In one embodiment, the top surface of the porous mesh 302 is covered by one or more removable top liners. In FIG. 10A, a center segment of a removable top liner has been removed (e.g., peeled away) so that the incision is visible thought the porous mesh. In FIG. 10A, first and second peripheral segments 320A, 320B of the removable top liner remain covering the top surface of the porous mesh 302. The first and second peripheral segments 320A, 320B may be engaged by a surgeon's fingers for manipulating the porous mesh 302 so that the porous mesh is properly aligned over the incision and/or for effectively approximating the opposing skin edges of the incision.

Referring to FIG. 10B, in one embodiment, after the porous mesh 302 has been aligned over the incision and has been configured for approximating the opposing skin edges of the incision, the peripheral segments of the removable top liner including the second peripheral segment 320B of the removable top liner may be peeled away from the top surface of the porous mesh 302.

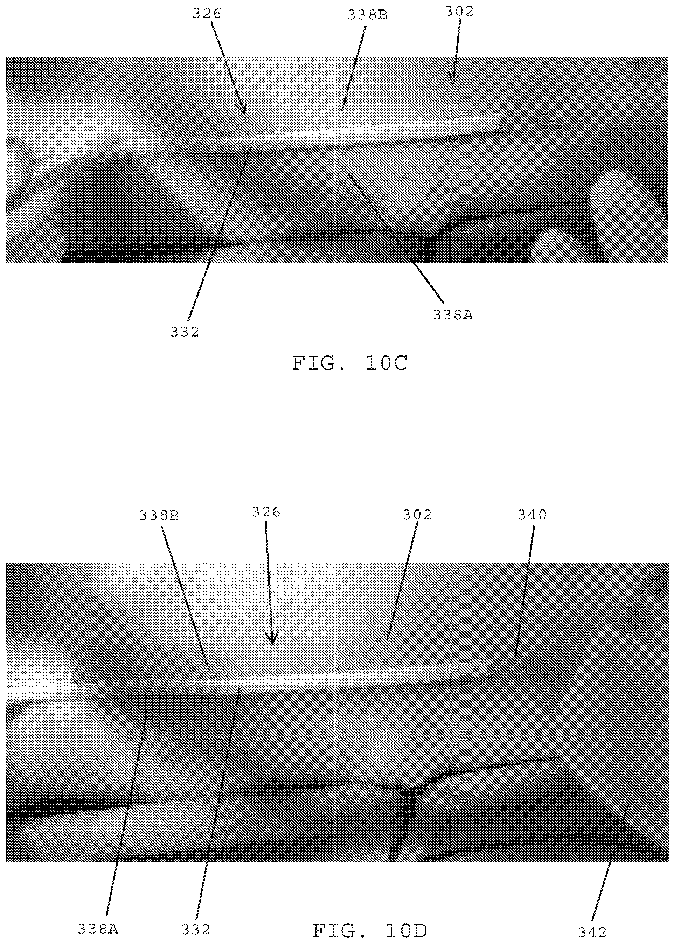

Referring to FIG. 10C, in one embodiment, when the center and first and second peripheral segments of the releasable top liner have been removed from covering the top surface of the porous mesh 302, the bottom surface of the porous mesh remains secured to the skin of the patient for approximating the opposing skin edges of the incision (FIG. 10B). The top surface of the porous mesh may be covered with a pressure sensitive adhesive (e.g., spaced traces of the pressure sensitive adhesive). In one embodiment, a therapeutic device 326 including an elongated tube 332 and first and second mesh wings 338A, 338B that extend along opposite lateral sides of the elongated tube is preferably positioned over the top surface of the porous mesh 302. The therapeutic device 326 may be similar to the therapeutic device 226 shown and describe above in FIGS. 9B-9E. In one embodiment, the elongated tube 332 is desirably aligned with the incision (FIG. 10B) so that the elongated tube 332 extends along the length of the incision.

Referring to FIG. 10D, in one embodiment, a liquid adhesive 340 is preferably applied over the therapeutic device 326, which includes the elongated tube 332 and the first and second mesh wings 338A, 338B. In addition, the liquid adhesive 340 is applied over the top surface of the porous mesh 302. In one embodiment, the liquid adhesive 340 preferably completely surrounds the outer periphery of the porous mesh 302 and encapsulates the therapeutic device 326 and the porous mesh 302. In one embodiment, an adhesive spreader 342 may be utilized for applying and/or spreading the liquid adhesive 340 over the therapeutic device 326 and the porous mesh 302.

In one embodiment, the liquid adhesive is configured to secure the therapeutic device 326 and the porous mesh over the incision and form a strong seal around the incision. The liquid adhesive may also form a microbial barrier, be waterproof, and/or allow medical personnel to monitor the wound healing process. The liquid adhesive may be any biocompatible adhesive known in the art, for example, a cyanoacrylate-based adhesive, a silicone-based adhesive, etc. as described above.

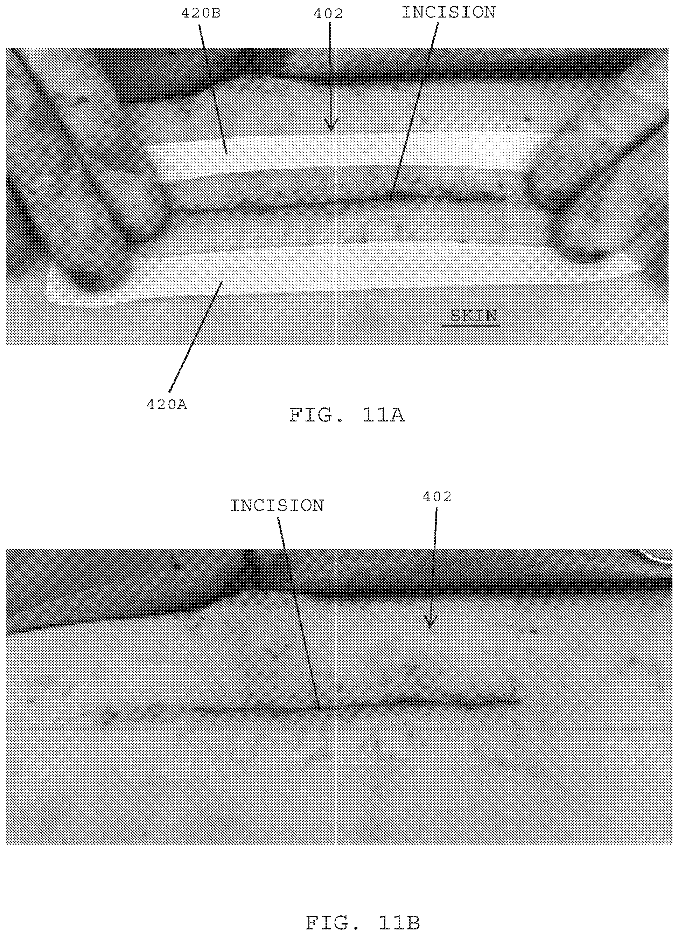

Referring to FIG. 11A, in one embodiment, a porous mesh 402 is preferably positioned over an incision formed in skin of a patient. A bottom surface of the porous mesh 402 may have an adhesive (e.g., a pressure sensitive adhesive) for adhering the bottom surface of the porous mesh to the skin of the patient. When the porous mesh 402 has been positioned over the incision, the porous mesh is preferably aligned with the incision and preferably approximates the opposing skin edges of the incision.

In one embodiment, the top surface of the porous mesh 402 is preferably covered by one or more removable top liners. In FIG. 11A, a center segment of the removable top liner has been removed so that the incision is visible through the porous mesh. In FIG. 11A, first and second peripheral segments 420A, 420B of the removable top liner remain in place over the top surface of the porous mesh 402. The first and second peripheral segments 420A, 420B may be gripped by a surgeon for controlling and/or manipulating the porous mesh 402 so that it may be properly aligned over the incision and/or for approximating the opposing skin edges of the incision.

Referring to FIG. 11B, in one embodiment, after the porous mesh 402 has been aligned over the incision, the peripheral segments 420A, 420B (FIG. 11A) of the removable top liner may be peeled away from the top surface of the porous mesh 402.

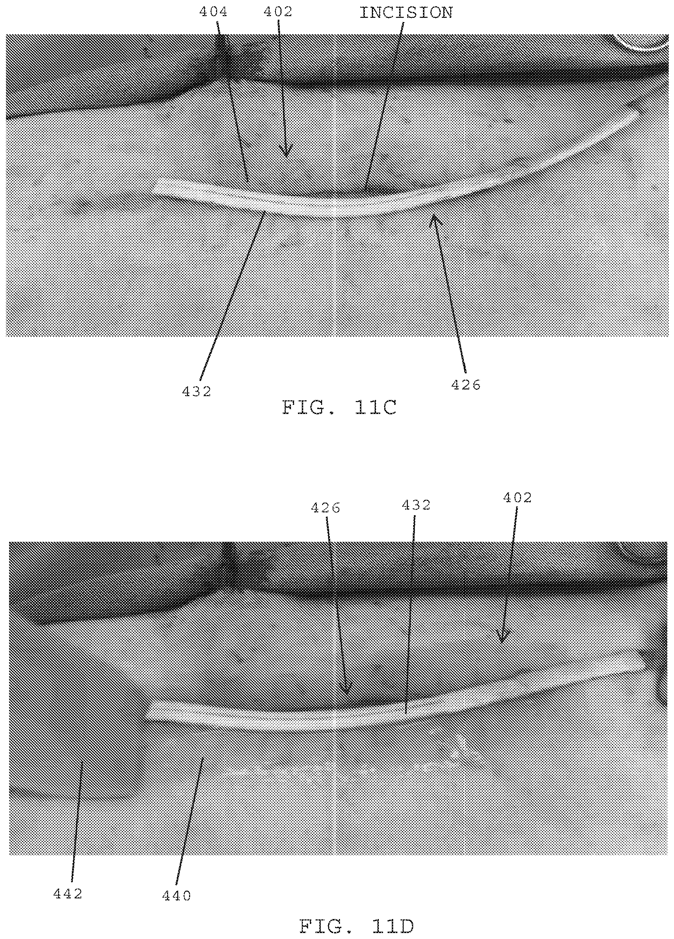

Referring to FIG. 11C, in one embodiment, when the center segment and the first and second peripheral segments of the releasable top liner have been removed from covering the top surface of the porous mesh 402, the bottom surface of the porous mesh remains secured to the skin of the patient for approximating the opposing skin edges of the incision. In one embodiment, a therapeutic device 426 including an elongated tube 432 is preferably positioned over the top surface 404 of the porous mesh 402. In one embodiment, the elongated tube 432 is desirably aligned with the incision (FIG. 11B) so that the elongated tube 432 extends along the length of the incision. The elongated tube 432 may be flexed into a shape (e.g., a curve) that mirrors and/or conforms to the shape of the underlying incision.

Referring to FIG. 11D, in one embodiment, a liquid adhesive 440 is preferably applied over the therapeutic device 426 including the elongated tube 432 and the top surface of the porous mesh 402. In one embodiment, an adhesive spreader 442 may be utilized for applying and/or spreading the liquid adhesive 440 over the therapeutic device 426 and the porous mesh 402. In one embodiment, the liquid adhesive 440 preferably completely surrounds the outer periphery of the porous mesh 402 and encapsulates the therapeutic device 426 and the porous mesh 402

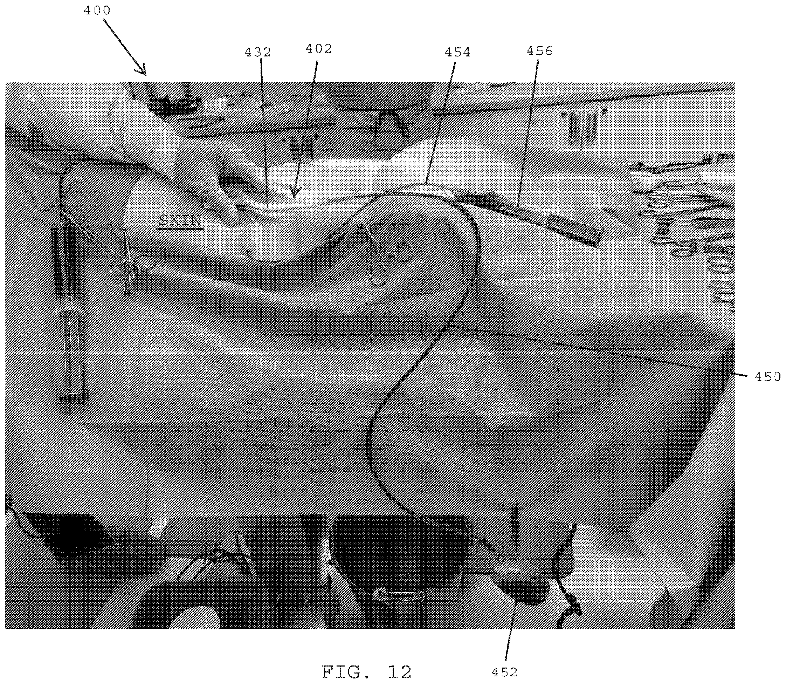

Referring to FIG. 12, in one embodiment, a wound closure system 400 preferably includes a therapeutic device including the elongated tube 432 secured atop a porous mesh 402 (FIG. 11B), which, in turn, is adhered to skin for approximating skin edges of the incision shown in FIGS. 11A-11D. The elongated tube 432 is preferably bifurcated into a first part that is coupled with a drainage tube 450 utilized for draining fluids from the incision. The drainage tube 450 may have a proximal end coupled with a fluid container 452 that collects the fluid (e.g., wound exudate) that passes through the drainage tube 450. In one embodiment, the wound closure system 400 preferably includes an infusion tube 454 coupled with a syringe 456 that may be utilized for infusing saline and/or a medication into the wound. After the saline or medication is infused into the wound, the fluid may be drawn from the wound via the drainage tube 450 for being collected within the collection container 452. The wound may be continuously flushed with fluids (e.g., saline; medication) for enhancing healing of the wound, which may include a combination of infusing the wound with saline and/or medication and draining wound exudate from the wound.

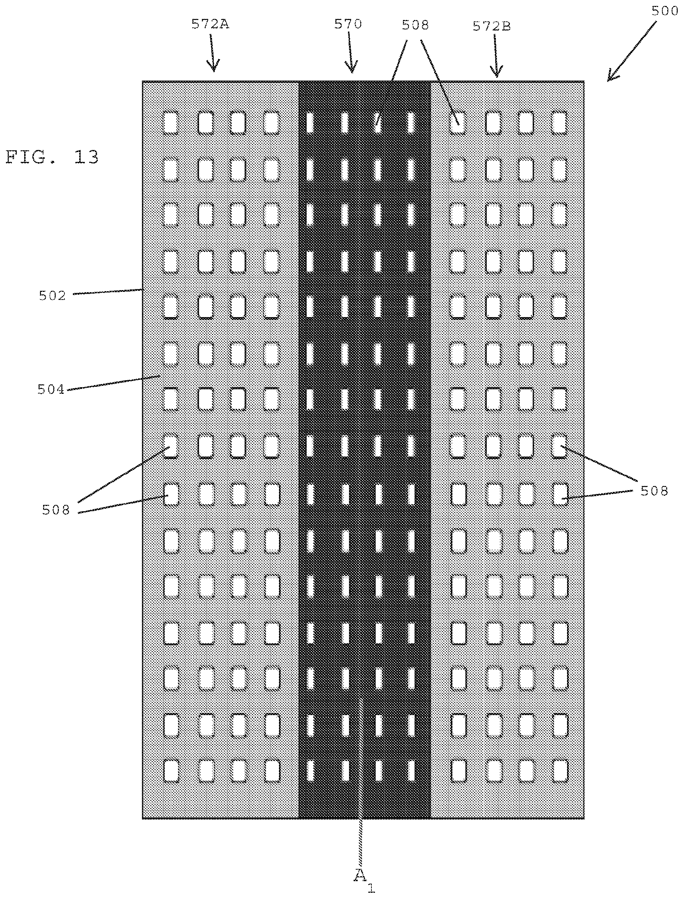

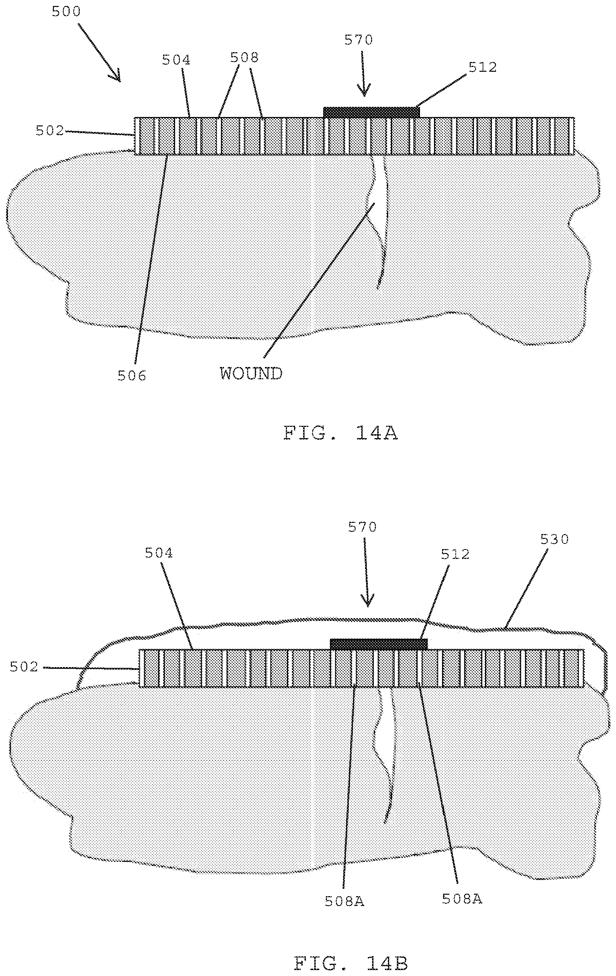

Referring to FIGS. 13 and 14A, in one embodiment, a wound closure system 500 preferably includes a porous mesh 502 having a top surface 504, a bottom surface 506, and a plurality of openings 508 (e.g., pores; holes) that extend between the top and bottom surfaces 504, 506. In one embodiment, the porous mesh 502 desirably includes a center section 570 that extends along a central axis A₁ and first and second peripheral sections 572A, 572B that extend along respective lateral sides of the center section 570. In one embodiment, the openings 508 located within the center section 570 of the porous mesh 502 have the same size as the openings 508 located within the first and second peripheral sections 572A, 572B of the porous mesh. In one embodiment, the openings 508 located within the center section 570 of the porous mesh 502 are smaller in size than the openings 508 located within the first and second peripheral sections 572A, 572B of the porous mesh.

Referring to FIG. 14A, in one embodiment, the bottom surface 506 of the porous mesh 502 is abutted against skin of a patient for approximating skin edges of a wound. The center section 570 of the porous mesh 502 that is covered by the releasable liner 512 is preferably aligned with the wound. The releasable liner 512 overlies the top surface 504 of the porous mesh 502 and covers the openings 508 located within the center section 570 of the porous mesh.

Referring to FIG. 14B, in one embodiment, a liquid adhesive 530 may be applied over the top surface 504 of the porous mesh 502 including the releasable liner 512 that covers the center section 570 of the porous mesh. The releasable liner 512 preferably prevents the liquid adhesive from closing of the openings 508A that are located within the center section 570 of the porous mesh 502. In one embodiment, in the stage shown in FIG. 14B, the liquid adhesive 530 preferably surrounds the outer perimeter of the porous mesh 502 and fully encapsulates the porous mesh and the releasable liner 512 that covers the center section 570 of the porous mesh. The releasable liner 512 preferably masks the openings 508A located within the center region 570 of the porous mesh.

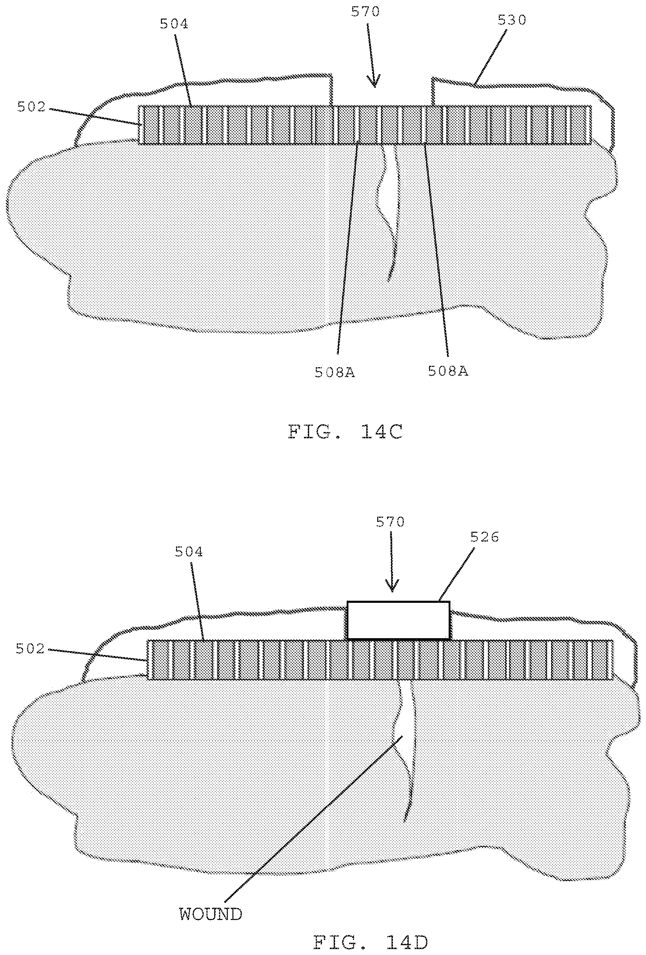

Referring to FIGS. 14B and 14C, in one embodiment, the liquid adhesive 130 is preferably not fully cured or only partially cured so that the releasable liner 512 (FIG. 14B) may be peeled away from the top surface 504 of the porous mesh 502 removing uncured or partially cured adhesive on the releasable liner 512 to expose the openings 508A that are located within the center section 570 of the porous mesh 502. The openings 508A within the center section 570 are located within a gap of the partially or fully cured adhesive 530. The gap in the adhesive desirably extends along the length of the center region of the porous mesh.

Referring to FIG. 14D, in one embodiment, after the releasable liner 512 (FIG. 14B) is peeled away from the top surface 504 of the porous mesh 502, a therapeutic device 526 (e.g., a negative pressure wound therapy device) may be positioned and/or secured over the center section 570 of the porous mesh for being aligned with the wound. In one embodiment, the therapeutic device 526 is preferably configured for removing and/or draining bodily fluids (e.g., exudate) from the wound. The therapeutic device 526 may also be configured for delivering saline and/or medication to the wound or for monitoring the wound (e.g., a sensor).

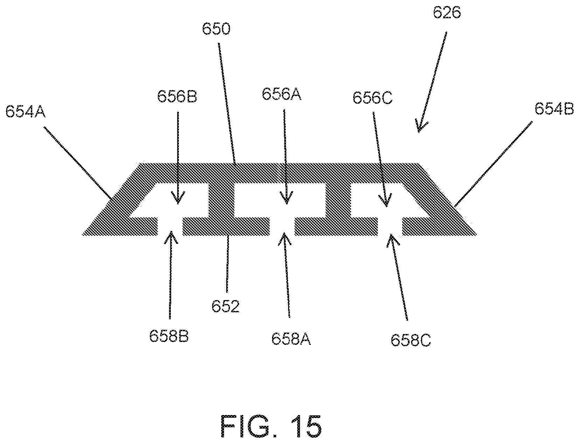

Referring to FIG. 15, in one embodiment, a therapeutic device 626 may be incorporated into one or more of the wound closure systems disclosed herein. In one embodiment, the therapeutic device 626 may be used for draining a wound during negative pressure wound therapy and/or for infusing a therapeutic solution into a wound. In one embodiment, the therapeutic device 626 may be used for both draining a wound and infusing the wound with a therapeutic solution.

In one embodiment, the therapeutic device 626 may be made of a biocompatible material such as a polymer, silicone, or stainless steel. In one embodiment, the therapeutic device may be transparent.

In one embodiment, the therapeutic device 626 preferably has a top surface 650, a bottom surface 652, and side walls 654A, 654B that slope outwardly between the top and bottom surfaces 652, 654. In one embodiment, the top and bottom surfaces 650, 652 of the therapeutic device may be parallel to one another. In one embodiment, the therapeutic device 626 may have a low profile and may resemble the shape of a speed bump.

In one embodiment, the therapeutic device 626 may have a first elongated channel 656A that extends along the length of the therapeutic device. In one embodiment, the therapeutic device 626 preferably includes a first fenestration 658A (e.g., an opening) that is formed in the bottom surface 652 of the therapeutic device, which is in fluid communication with the first elongated channel 656A. In one embodiment, the first fenestration 658A may be positioned over a wound for placing the first elongated channel 656A in fluid communication with the wound. In one embodiment, a vacuum may be coupled with the first elongated channel 656A for draining fluid (e.g., exudate) from the wound. The fluid that is drained from the wound preferably passes through the first fenestration 658A and into the first elongated channel 656A for being removed from the wound. In one embodiment, the therapeutic device may include a first port that is in fluid communication with the first elongated channel 656A for coupling a drain tube with the first elongated channel 656A.

In one embodiment, the first elongated channel 656A and the first fenestration 658A may be used for infusing a therapeutic solution into the wound.

In one embodiment, the therapeutic device 626 may have a second elongated channel 656B that extends along the length of the therapeutic device and a second fenestration 658B formed in the bottom surface 652 of the therapeutic device that is in fluid communication with the second elongated channel 656B.

In one embodiment, the second elongated channel 656B and the second fenestration 658B may be used for draining fluid from a wound and/or for infusing the wound with a therapeutic solution. In one embodiment, the therapeutic device may include a second port that is in fluid communication with the second elongated channel 656B for coupling the second elongated channel with a drainage tube or an infusion tube.

In one embodiment, the therapeutic device 626 may have a third elongated channel 656C that extends along the length of the therapeutic device and a third fenestration 658C formed in the bottom surface 652 of the therapeutic device that is in fluid communication with the third elongated channel 656C.

In one embodiment, the third elongated channel 656C and the third fenestration 658C may be used for draining fluid from a wound and/or for infusing a therapeutic solution into the wound. In one embodiment, the therapeutic device may include a third port that is in fluid communication with the third elongated channel 656C for coupling the third elongated channel with a drainage tube or an infusion tube.

In one embodiment, the therapeutic device 626 may be secured over a top surface of a first layer of a porous dressing so that the first elongated channel 656A of the therapeutic device is positioned over the top surface of the first layer of the porous dressing with the first elongated channel positioned adjacent the wound.

In one embodiment, a second layer of a porous dressing may be applied over both the first layer of the porous dressing and over the top of the therapeutic device 626 to secure the therapeutic device to the top surface of the first layer of the porous dressing. The second layer of the porous dressing may contact the top surface 650 and the side surfaces 654A, 654B of the therapeutic device 626.

In one embodiment, a flowable adhesive may be applied over the first layer of the porous dressing, the second layer of the porous dressing and the therapeutic device 626. The flowable adhesive may be cured for forming an air-tight seal around the wound.

In one embodiment, a tube may be attached to the therapeutic device 626 for draining fluid (e.g., exudate) from the first elongated channel 656A of the therapeutic device. In one embodiment, the tube may be attached to a vacuum for applying negative pressure to the wound for draining fluid from the wound. In one embodiment, the tube may be attached to a therapeutic solution (e.g., saline; medication; an antibiotic solution) for infusing the therapeutic solution into the first elongated channel 656A. The therapeutic solution preferably passes through the first fenestration 658A for infusing the wound with the therapeutic solution.

In one embodiment, the elongated channel of the therapeutic device 656 desirably has two or more connection ports where fluid may be infused into and/or withdrawn from the wound.

In one embodiment, the connection ports may be located on the top surface 650 or on one or more of the side surfaces 654A, 654B of the therapeutic device 626. In one embodiment, a connection port may be attached to a vacuum via tubing, which is configured to apply negative pressure to a wound or incision. In one embodiment, a connection port may be attached to an infusing tube for infusing a therapeutic solution (e.g., saline; medication; antibiotic) into a wound or incision.

In one embodiment, the therapeutic device 626 may be transparent for enabling medical personnel to view the wound or incision during healing. In one embodiment, the flowable adhesive cures to a transparent covering for enabling medical personnel to view the wound or incision during healing. In one embodiment, the flowable adhesive is a silicone material.

While the foregoing is directed to embodiments of the present invention, other and further embodiments of the invention may be devised without departing from the basic scope thereof, which is only limited by the scope of the claims that follow.

## Claims

1. A wound closure system (100, 200, 400, 500) comprising:
a porous mesh (102, 202, 302, 402, 502) having a top surface (104, 504) and a bottom surface (106, 506);
a first pressure sensitive adhesive (110) applied to the top surface (104) of said porous mesh;
a removable first liner (112, 512) covering said first pressure sensitive adhesive (110);
a second pressure sensitive adhesive (114) applied to the bottom surface (106) of said porous mesh;
a removable second liner (116) covering said second pressure sensitive adhesive (114);
said second pressure sensitive adhesive (114) being stronger or denser than said first pressure sensitive adhesive (110),
a therapeutic device (126, 226, 326, 426, 526) including an elongated tube (128, 232, 332, 432) secured to the top surface of said porous mesh, said elongated tube being configured for draining exudate from a wound; and
a liquid adhesive (130, 240, 340, 440, 530) covering the top surface of said porous mesh and at least a portion of said elongated tube.

2. The wound closure system (100, 200, 400, 500) as claimed in claim 1, further comprising a cross-linking or polymerization accelerator or catalyst disposed in or on said porous mesh (102, 202, 302, 402, 502).

3. The wound closure system (100, 200, 400, 500) as claimed in claim 1 or claim 2, wherein said porous mesh (102, 202, 302, 402, 502) is flat, elongated along a longitudinal axis, and has a plurality of openings (108, 508) extending between the top and bottom surfaces (104, 106) thereof.

4. The wound closure system (100, 200, 400, 500) as claimed in any preceding claim, wherein said removable first liner (112) is configured to be peeled away from the top surface (104) of said porous mesh for exposing said first pressure sensitive adhesive (110), and wherein said removable second liner (116) is configured to be peeled away from the bottom surface (106) of said porous mesh for exposing said second pressure sensitive adhesive (114) .

5. The wound closure system (100, 200, 400, 500) as claimed in any preceding claim, said therapeutic device (126, 226, 326, 426, 526) further comprising first and second mesh wings (238A, 238B) secured to said elongated tube (128, 232, 332, 432) and extending along respective lateral sides of said elongated tube, wherein said liquid adhesive (130, 240, 340, 440, 530) covers said first and second mesh wings for securing said therapeutic device to the top surface of said porous mesh (102, 202, 302, 402, 502).

6. The wound closure system (100, 200, 400, 500) as claimed in any preceding claim, wherein said elongated tube (128, 232, 332, 432) comprises a first elongated conduit (246) that is coupled with a vacuum or negative pressure pump (244) for draining exudate from a wound.

7. The wound closure system (100, 200, 400, 500) as claimed in any preceding claim, wherein said elongated tube (128, 232, 332, 432) comprises a second elongated conduit (248) that is coupled with a positive pressure pump (244) configured to force saline or medication into said elongated tube.

8. The wound closure system (100, 200, 400, 500) as claimed in any preceding claim, wherein said liquid adhesive (130, 240, 340, 440, 530) is a topical skin adhesive or a rapidly polymerizable adhesive suitable for forming an air-tight seal around a wound, optionally, wherein the air-tight seal formed by said liquid adhesive is waterproof or comprises an antimicrobial barrier.

9. A kit comprising:
a wound closure system (100) as claimed in any one of claims 1 to 4;
a rapidly polymerizable liquid adhesive (130, 240, 340, 440, 530) disposed within a dispenser container; and
one or more therapeutic devices (126, 226, 326, 426, 526) configured to be secured over the top surface of said porous mesh (102, 202, 302, 402, 502).

10. The kit as claimed in claim 9, wherein said one or more therapeutic devices (126, 226, 326, 426, 526) are selected from the group of devices consisting of negative pressure wound therapy devices, negative pressure pumps, fluid suction devices, elongated tubes (128, 232, 332, 432) including drainage tubes and infusion tubes, elongated tubes (128, 232, 332, 432) having first and second mesh wings (238A, 238B) extending along lateral sides thereof, cannulas, and wound monitoring sensors.

11. The kit as claimed in claim 9, wherein the therapeutic device (126, 226, 326, 426, 526) includes an elongated tube (128, 232, 332, 432), said elongated tube being suitable for draining exudate from a wound.

12. The kit as claimed in claim 11, said therapeutic device (126, 226, 326, 426, 526) further comprising first and second mesh wings secured to said elongated tube (128, 232, 332, 432) and extending along respective lateral sides of said elongated tube.

13. The kit as claimed in claim 11 or claim 12, wherein said elongated tube (128, 232, 332, 432) comprises a first elongated conduit (246) that is coupled with a vacuum or negative pressure pump (244) suitable for draining exudate from a wound.

14. The kit as claimed in any one of claim 11 to 13, wherein said elongated tube (128, 232, 332, 432) comprises a second elongated conduit (248) that is coupled with a positive pressure pump (244) configured to force saline or medication into said elongated tube.

## Patentansprüche

1. Wundverschlusssystem (100, 200, 400, 500), umfassend:
ein poröses Netz (102, 202, 302, 402, 502), das eine obere Oberfläche (104, 504) und eine untere Oberfläche (106, 506) aufweist;
einen ersten Haftklebstoff (110), der auf die obere Oberfläche (104) des porösen Netzes aufgebracht wird;
eine entfernbare erste Trennschicht (112, 512), die den ersten Haftklebstoff (110) bedeckt;
einen zweiten Haftklebstoff (114), der auf die untere Oberfläche (106) des porösen Netzes aufgebracht ist;
eine entfernbare zweite Trennschicht (116), die den zweiten Haftklebstoff (114) bedeckt;
wobei der zweite Haftklebstoff (114) stärker oder dichter als der erste Haftklebstoff (110) ist,
eine therapeutische Vorrichtung (126, 226, 326, 426, 526), die einen länglichen Schlauch (128, 232, 332, 432) einschließt, der an der oberen Oberfläche des porösen Netzes befestigt ist, wobei der längliche Schlauch zum Ableiten von Exsudat aus einer Wunde konfiguriert ist; und
einen flüssigen Klebstoff (130, 240, 340, 440, 530), der die obere Oberfläche des porösen Netzes und mindestens einen Abschnitt des länglichen Schlauchs bedeckt.

2. Wundverschlusssystem (100, 200, 400, 500) nach Anspruch 1, ferner umfassend einen Vernetzungs- oder Polymerisationsbeschleuniger oder -katalysator, der in oder auf dem porösen Netz (102, 202, 302, 402, 502) angeordnet ist.

3. Wundverschlusssystem (100, 200, 400, 500) nach Anspruch 1 oder 2, wobei das poröse Netz (102, 202, 302, 402, 502) flach, entlang einer Längsachse langgestreckt ist und eine Vielzahl von Öffnungen (108, 508) aufweist, die sich zwischen der oberen und der unteren Oberfläche (104, 106) davon erstrecken.

4. Wundverschlusssystem (100, 200, 400, 500) nach einem der vorstehenden Ansprüche, wobei die entfernbare erste Trennfolie (112) konfiguriert ist, um von der oberen Oberfläche (104) des porösen Netzes zum Freilegen des ersten Haftklebstoffs (110) abgezogen zu werden, und wobei die entfernbare zweite Trennfolie (116) konfiguriert ist, um von der unteren Oberfläche (106) des porösen Netzes zum Freilegen des zweiten Haftklebstoffs (114) abgezogen zu werden.

5. Wundverschlusssystem (100, 200, 400, 500) nach einem vorstehenden Anspruch, die therapeutische Vorrichtung (126, 226, 326, 426, 526) ferner umfassend einen ersten und einen zweiten Netzflügel (238A, 238B), die an dem länglichen Schlauch (128, 232, 332, 432) befestigt sind und sich entlang jeweiliger lateraler Seiten des länglichen Schlauchs erstrecken, wobei der flüssige Klebstoff (130, 240, 340, 440, 530) den ersten und den zweiten Netzflügel bedeckt, um die therapeutische Vorrichtung an der oberen Oberfläche des porösen Netzes (102, 202, 302, 402, 502) zu befestigen.

6. Wundverschlusssystem (100, 200, 400, 500) nach einem vorstehenden Anspruch, wobei der längliche Schlauch (128, 232, 332, 432) eine erste längliche Leitung (246) umfasst, die mit einer Vakuum- oder Unterdruckpumpe (244) zum Ableiten von Exsudat aus einer Wunde gekoppelt ist.

7. Wundverschlusssystem (100, 200, 400, 500) nach einem der vorstehenden Ansprüche, wobei der längliche Schlauch (128, 232, 332, 432) eine zweite längliche Leitung (248) umfasst, die mit einer Überdruckpumpe (244) gekoppelt ist, die konfiguriert ist, um Kochsalzlösung oder Medikamente in den länglichen Schlauch zu pressen.

8. Wundverschlusssystem (100, 200, 400, 500) nach einem der vorstehenden Ansprüche, wobei der flüssige Klebstoff (130, 240, 340, 440, 530) ein topischer Hautklebstoff oder ein schnell polymerisierbarer Klebstoff ist, der zum Ausbilden einer luftdichten Abdichtung um eine Wunde herum geeignet ist, gegebenenfalls wobei die luftdichte Abdichtung, die durch den flüssigen Klebstoff ausgebildet wird, wasserdicht ist oder eine antimikrobielle Barriere umfasst.

9. Kit, umfassend:
ein Wundverschlusssystem (100) wie in einem der Ansprüche 1 bis 4 beansprucht;
einen schnell polymerisierbaren flüssigen Klebstoff (130, 240, 340, 440, 530), der innerhalb eines Spenderbehälters angeordnet ist; und
eine oder mehrere therapeutische Vorrichtungen (126, 226, 326, 426, 526), die konfiguriert sind, um über der oberen Oberfläche des porösen Netzes (102, 202, 302, 402, 502) befestigt zu werden.

10. Kit nach Anspruch 9, wobei die eine oder die mehreren therapeutischen Vorrichtungen (126, 226, 326, 426, 526) aus der Gruppe von Vorrichtungen ausgewählt sind, bestehend aus Unterdruckwundtherapievorrichtungen, Unterdruckpumpen, Fluidabsaugvorrichtungen, länglichen Schläuchen (128, 232, 332, 432), einschließlich Ableitungsschläuchen und Infusionsschläuchen, länglichen Schläuchen (128, 232, 332, 432), die den ersten und den zweiten Netzflügel (238A, 238B) aufweisen, die sich entlang lateralen Seiten davon erstrecken, Kanülen und Wundüberwachungssensoren.

11. Kit nach Anspruch 9, wobei die therapeutische Vorrichtung (126, 226, 326, 426, 526) einen länglichen Schlauch (128, 232, 332, 432) einschließt, wobei der längliche Schlauch zum Ableiten von Exsudat aus einer Wunde geeignet ist.

12. Kit nach Anspruch 11, die therapeutische Vorrichtung (126, 226, 326, 426, 526) ferner umfassend den ersten und den zweiten Netzflügel, die an dem länglichen Schlauch (128, 232, 332, 432) befestigt sind und sich entlang jeweiliger lateraler Seiten des länglichen Schlauchs erstrecken.

13. Kit nach Anspruch 11 oder 12, wobei der längliche Schlauch (128, 232, 332, 432) eine erste längliche Leitung (246) umfasst, die mit einer Vakuum- oder Unterdruckpumpe (244) gekoppelt ist, die zum Ableiten von Exsudat aus einer Wunde geeignet ist.

14. Kit nach einem der Ansprüche 11 bis 13, wobei der längliche Schlauch (128, 232, 332, 432) eine zweite längliche Leitung (248) umfasst, die mit einer Überdruckpumpe (244) gekoppelt ist, die konfiguriert ist, um Kochsalzlösung oder Medikamente in den länglichen Schlauch zu pressen.

## Revendications

1. Système de fermeture de plaie (100, 200, 400, 500) comprenant :
une maille poreuse (102, 202, 302, 402, 502) ayant une surface supérieure (104, 504) et une surface inférieure (106, 506) ;
un premier adhésif sensible à la pression (110) appliqué sur la surface supérieure (104) de ladite maille poreuse ;
un premier revêtement amovible (112, 512) recouvrant ledit premier adhésif sensible à la pression (110) ;
un second adhésif sensible à la pression (114) appliqué sur la surface inférieure (106) de ladite maille poreuse ;
un second revêtement amovible (116) recouvrant ledit second adhésif sensible à la pression (114) ;
ledit second adhésif sensible à la pression (114) étant plus résistant ou plus dense que ledit premier adhésif sensible à la pression (110),
un dispositif thérapeutique (126, 226, 326, 426, 526) comportant un tube allongé (128, 232, 332, 432) fixé à la surface supérieure de ladite maille poreuse, ledit tube allongé étant conçu pour drainer un exsudat d'une plaie ; et
un adhésif liquide (130, 240, 340, 440, 530) recouvrant la surface supérieure de ladite maille poreuse et au moins une partie dudit tube allongé.

2. Système de fermeture de plaie (100, 200, 400, 500) selon la revendication 1, comprenant en outre un accélérateur ou un catalyseur de réticulation ou de polymérisation disposé dans ou sur ladite maille poreuse (102, 202, 302, 402, 502).

3. Système de fermeture de plaie (100, 200, 400, 500) selon la revendication 1 ou la revendication 2, dans lequel ladite maille poreuse (102, 202, 302, 402, 502) est plate, allongée le long d'un axe longitudinal, et a une pluralité d'ouvertures (108, 508) s'étendant entre les surfaces supérieure et inférieure (104, 106) de celle-ci.

4. Système de fermeture de plaie (100, 200, 400, 500) selon l'une quelconque revendication précédente, dans lequel ledit premier revêtement amovible (112) est conçu pour être décollé de la surface supérieure (104) de ladite maille poreuse pour exposer ledit premier adhésif sensible à la pression (110), et dans lequel ledit second revêtement amovible (116) est conçu pour être décollé de la surface inférieure (106) de ladite maille poreuse pour exposer ledit second adhésif sensible à la pression (114).

5. Système de fermeture de plaie (100, 200, 400, 500) selon l'une quelconque revendication précédente, ledit dispositif thérapeutique (126, 226, 326, 426, 526) comprenant en outre des première et seconde ailettes en maille (238A, 238B) fixées audit tube allongé (128, 232, 332, 432) et s'étendant le long de côtés latéraux respectifs dudit tube allongé, dans lequel ledit adhésif liquide (130, 240, 340, 440, 530) recouvre lesdites première et seconde ailettes en maille pour fixer ledit dispositif thérapeutique à la surface supérieure de ladite maille poreuse (102, 202, 302, 402, 502).

6. Système de fermeture de plaie (100, 200, 400, 500) selon l'une quelconque revendication précédente, dans lequel ledit tube allongé (128, 232, 332, 432) comprend un premier conduit allongé (246) qui est accouplé à une pompe à vide ou à pression négative (244) pour drainer l'exsudat d'une plaie.

7. Système de fermeture de plaie (100, 200, 400, 500) selon l'une quelconque revendication précédente, dans lequel ledit tube allongé (128, 232, 332, 432) comprend un second conduit allongé (248) qui est accouplé à une pompe à pression positive (244) conçue pour forcer une solution saline ou un médicament à entrer dans ledit tube allongé.

8. Système de fermeture de plaie (100, 200, 400, 500) selon l'une quelconque revendication précédente, dans lequel ledit adhésif liquide (130, 240, 340, 440, 530) est un adhésif cutané topique ou un adhésif à polymérisation rapide approprié pour former un joint étanche à l'air autour d'une plaie, éventuellement, dans lequel le joint étanche à l'air formé par ledit adhésif liquide est imperméable à l'eau ou comprend une barrière antimicrobienne.

9. Trousse comprenant :
un système de fermeture de plaie (100) selon l'une quelconque des revendications 1 à 4 ;
un adhésif liquide à polymérisation rapide (130, 240, 340, 440, 530) disposé à l'intérieur d'un récipient distributeur ; et
un ou plusieurs dispositifs thérapeutiques (126, 226, 326, 426, 526) conçus pour être fixés sur la surface supérieure de ladite maille poreuse (102, 202, 302, 402, 502).

10. Trousse selon la revendication 9, dans laquelle ledit ou lesdits dispositifs thérapeutiques (126, 226, 326, 426, 526) sont choisis dans le groupe de dispositifs constitué de dispositifs de traitement de plaies par pression négative, pompes à pression négative, dispositifs d'aspiration de fluide, tubes allongés (128, 232, 332, 432) comportant des tubes de drainage et des tubes de perfusion, tubes allongés (128, 232, 332, 432) ayant des première et seconde ailettes en maille (238A, 238B) s'étendant le long de leurs côtés latéraux, canules et capteurs de surveillance de plaies.

11. Trousse selon la revendication 9, dans laquelle le dispositif thérapeutique (126, 226, 326, 426, 526) comporte un tube allongé (128, 232, 332, 432), ledit tube allongé étant approprié pour drainer un exsudat d'une plaie.

12. Trousse selon la revendication 11, ledit dispositif thérapeutique (126, 226, 326, 426, 526) comprenant en outre des première et seconde ailettes en maille fixées audit tube allongé (128, 232, 332, 432) et s'étendant le long de côtés latéraux respectifs dudit tube allongé.

13. Trousse selon la revendication 11 ou la revendication 12, dans laquelle ledit tube allongé (128, 232, 332, 432) comprend un premier conduit allongé (246) qui est accouplé à une pompe à vide ou à pression négative (244) appropriée pour drainer l'exsudat d'une plaie.

14. Trousse selon l'une quelconque des revendications 11 à 13, dans laquelle ledit tube allongé (128, 232, 332, 432) comprend un second conduit allongé (248) qui est accouplé à une pompe à pression positive (244) conçue pour forcer une solution saline ou un médicament à entrer dans ledit tube allongé.
